# EUROPÄISCHE PATENTSCHRIFT

(11) **EP 2 454 296 B1**
(45) Veröffentlichungstag und Bekanntmachung des Hinweises auf die Patenterteilung: **11.11.2015**
(21) Anmeldenummer: 10730180.6
(22) Anmeldetag: 09.07.2010
(51) Int. Cl.: C08F 220/06, C08F 220/26, C07C 57/00

(54) **COPOLYMERE, IHRE VERWENDUNG ALS VERDICKER, UND VERFAHREN ZU IHRER HERSTELLUNG**
COPOLYMERS, THEIR USE AS THICKENERS AND METHOD FOR PRODUCING THE SAME
COPOLYMÈRES, LEUR UTILISATION COMME ÉPAISSISSANTS ET PROCÉDÉ POUR LEUR PRODUCTION

(30) Priorität: 15.07.2009 EP 09165576
(43) Veröffentlichungstag der Anmeldung: 23.05.2012
(73) Patentinhaber: BASF SE, 67056 Ludwigshafen (DE)
(72) Erfinder: AL-HELLANI, Rabie, 67063 Ludwigshafen (DE); BRUCHMANN, Bernd, 67251 Freinsheim (DE); SCHÖNFELDER, Daniel, B-1000 Bruxelles (BE); CRISTADORO, Anna, 64646 Heppenheim (DE); LEYRER, Reinhold J, 67125 Dannstadt-Schauernheim (DE); ARISANDY, Christofer, 68549 Ilvesheim (DE)
(86) Internationale Anmeldenummer: PCT/EP2010/059848
(87) Internationale Veröffentlichungsnummer: WO 2011/006838

(56) Entgegenhaltungen:
- WO-A1-99/65958

## Beschreibung

Die vorliegende Erfindung betrifft Copolymere, enthaltend als Comonomer einpolymerisiert
(A) mindestens eine ethylenisch ungesättigte Mono- oder Dicarbonsäure,
(B) mindestens ein (Co)polymer, das pro Molekül mindestens eine Struktureinheit der allgemeinen Formel I aufweist: 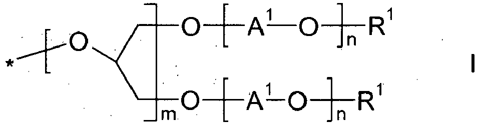 wobei die Variablen wie folgt definiert sind:
   - R¹: verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
   - A¹: verschieden oder gleich und gewählt aus C₂-C₁₀-Alkylen, C₆-C₁₀-Arylen und C₇-C₁₀-Aralkylen,
   - n: verschieden oder gleich und gewählt aus null bis 200,
   - m: verschieden oder gleich und gewählt aus 1 bis 6,
(C) mindestens ein weiteres Comonomer.

Weiterhin betrifft die vorliegende Erfindung die Verwendung von erfindungsgemäßen Copolymeren als Verdicker, beispielsweise in Wasch- oder Reinigungsmitteln.

Weiterhin betrifft die vorliegende Erfindung Verbindungen (B), die als Comonomer zur Herstellung von erfindungsgemäßen Copolymer besonders geeignet sind. Weiterhin betrifft die vorliegende Erfindung Verbindungen, die zur Synthese von erfindungsgemäßen (Co)polymeren besonders geeignet sind.

Um in wässrigen Formulierungen, beispielsweise Wasch- und Reinigungsmitteln, in Druckpasten oder in Körperpflegemitteln und kosmetischen Zubereitungen die Viskosität einzustellen, verwendet man vielfach Verdicker (Verdickungsmittel). Bei Verdickern kann es sich um natürliche oder synthetische Verdicker handeln. Bekannte synthetische Verdicker sind in vielen Fällen mit Ammoniak neutralisierte Copolymere von Acrylsäure mit Acrylamid, die geringe Mengen eines zweifach ethylenisch ungesättigten Comonomers wie beispielsweise Methylenbisacrylamid einpolymerisiert enthalten.

Die Eigenschaften von bekannten Verdickern lassen sich noch verbessern.

Unerwünscht ist in vielen Fällen das Weißöl, welches für die Synthese der Verdicker erforderlich ist, sich somit zumindest anteilig hoch in den betreffenden synthetischen Verdickern befindet und das in Anwendungen auf faserigen Materialien wie beispielsweise Textil zurückbleibt und dort zu verschlechterten Echtheiten und zu einem verschlechterten Griff führen kann. Insbesondere dann, wenn man Druckpasten auf Basis von Dispersfarbstoffen (Dispersionsfarbstoffen) oder Reaktivfarbstoffen einsetzen möchte, ist es erwünscht, den Verdicker durch einfaches Waschen zu entfernen, was bei der Verwendung von Weißöl-haltigen Verdickern in vielen Fällen nicht gelingt.

Man hat auch versucht, das Weißöl nach der Synthese des Verdickers zu entfernten, beispielsweise durch Trocknung des Verdickers, insbesondere durch Sprühtrocknung. Man erhält dann so genannte Pulververdicker, die sich aber nicht stets in andere Formulierungen gut einrühren lassen.

Man hat versucht, die Nachteile des Weißöls durch die Verwendung von Assoziativerdickern zu umgehen. Assoziativverdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen in Wechselwirkung zu treten, insbesondere zu assoziieren und ein Netzwerk zu bilden. Durch das resultierende assoziative Netzwerk wird das Medium verdickt oder geliert.

Bekannte Assoziativverdicker lassen sich jedoch noch verbessern. Insbesondere die Salzstabilität ist in vielen Fällen noch nicht ausreichend. Außerdem ist im Falle von Flüssigwaschmitteln in vielen Fällen eine verbesserte Transparenz wünschenswert, die einen besseren Verkaufserfolg ermöglicht.

WO 99/65958 beschreibt alkalilösliche Verdicker, die das Umsetzungsprodükt einer ungesättigten Carbonsäure, eines monoethylenisch ungesättigten Monomers und eines hydrophoben, alkoxylierten Macromonomers umfassen. Das monoethylenisch ungesättigte Monomer umfasst eine Methylgruppe; vorzugsweise handelt es sich um Methylacrylat. Diese Polymere sollen bereits bei einem pH-Wert im Bereich von 4,5 bis 6,0 wasserlöslich werden und sich daher für kosmetische Erzeugnisse eignen.

Es bestand also die Aufgabe, Waschmittel bereit zu stellen, die ein verbessertes Waschverhalten bei hohem Salzgehalt und eine verbesserte Transparenz aufweisen. Es bestand weiterhin die Aufgabe, Zusätze für Waschmittel bereit zu stellen, mit deren Hilfe sich die gewünschten Eigenschaften einstellen lassen. Es bestand weiterhin die Aufgabe, ein Verfahren zur Herstellung der betreffenden Zusätze bereit zu stellen. Dementsprechend wurden die eingangs definierten Copolymere gefunden, kurz auch als erfindungsgemäße Copolymere bezeichnet. Erfindungsgemäße Copolymere enthalten als Comonomere einpolymerisiert
(A) mindestens eine ethylenisch ungesättigte Mono- oder Dicarbonsäure,
(B) mindestens ein (Co)polymer, das unten näher beschrieben wird und das kurz auch als Verbindung (B) oder erfindungsgemäßes Verbindung (B) bezeichnet wird,
(C) mindestens ein weiteres Comonomer.

Als ethylenisch ungesättigte Mono- oder Dicarbonsäure (A) sind beispielsweise mono- und mehrfach ethylenisch ungesättigte Mono- oder Dicarbonsäuren, beispielsweise Sorbinsäure, und insbesondere monoethylenisch ungesättigte C₃-C₁₀-Monocarbonäuren, monoethylenisch ungesättigte C₄-C₁₀-Dicarbonsäuren und ihre niedermolekularen Anhydride geeignet. Beispiele für geeignete monoethylenisch ungesättigte C₄-C₁₀-Dicarbonsäuren und ihre niedermolekularen Anhydride sind Itaconsäure, Citraconsäure, Mesaconsäure, Glutaconsäure, Aconitsäure, Maleinsäure, Fumarsäure, Maleinsäureanhydrid, Itaconsäureanhydrid und Mischungen von mehreren der vorstehend genannten Verbindungen. Beispiele für geeignete monoethylenisch ungesättigte C₃-C₁₀-Monocarbonäuren sind Ethacrylsäure, (E)- und (Z)-Crotonsäure und bevorzugt Acrylsäure. Besonders bevorzugt ist Methacrylsäure.

In einer Variante der vorliegenden Erfindung setzt man Mischungen von einer monoethylenisch ungesättigte C₃-C₁₀-Monocarbonäure und einer monoethylenisch ungesättigte C₄-C₁₀-Dicarbonsäure oder ihrem Anhydrid ein, beispielsweise von (Meth)acrylsäure und Maleinsäureanhydrid.

Erfindungsgemäße Copolymere enthalten weiterhin mindestens ein Verbindung (B) einpolymerisiert. Verbindungen (B) weisen pro Molekül mindestens eine, beispielsweise im Mittel 1 bis 10 (Zahlenmittel), bevorzugt bis 5 und besonders bevorzugt bis 2 Struktureinheiten der allgemeinen Formel I auf, 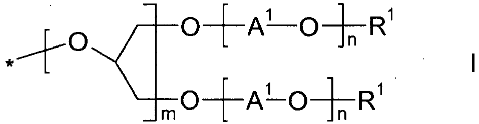 wobei die Variablen wie folgt definiert sind:
- R¹: verschieden oder vorzugsweise gleich und gewählt aus Wasserstoff und bevorzugt C₆-C₃₀-Aryl, bevorzugt C₆-C₁₄-Aryl, beispielsweise Phenyl, 1-Naphthyl, 2-Naphthyl, 1-Anthracenyl, 2-Anthracenyl, 9-Anthracenyl, C₇-C₃₀-Aralkyl, bevorzugt Benzyl,
C₃-C₁₀-Cycloalkyl, substituiert oder unsubstituiert, bevorzugt Cyclopentyl, Cyclohexyl, Cyclooctyl, wobei ein oder mehrere nicht-benachbarte C-Atome durch Sauerstoff oder N-H oder N-CH₃ ersetzt sein können, beispielhaft seien 1,3-Dioxolanyl, 1,3-Dioxanyl oder 13-Oxazolinyl genannt, bevorzugt sind 2,2-Dimethyl-1,3-dioxolanyl und 2,2-Dimethyl-1,3-dioxanyl genannt,
und insbesondere C₁-C₃₀-Alkyl, bevorzugt C₁-C₂₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl, n-Dodecyl, iso-Dodecyl, n-Tetradecyl, iso-Tetradecyl, n-Hexadecyl, iso-Hexadecyl, n-Octadecyl, iso-Octadecyl, n-Eicosyl, iso-Eicosyl.
- A¹: verschieden oder vorzugsweise gleich und gewählt aus C₆-C₁₀-Arylen, beispielsweise ortho-Phenylen, meta-Phenylen, para-Phenylen, 1,6-Naphthylen, 1,7-Naphthylen, 2,6-Naphthylen, 2,7-Naphthylen oder 1,8-Naphthylen, C₇-C₁₀-Aralkylen wie beispielsweise -CH(C₆H₅)- oder -CH₂-CH(C₆H₅)-,
und insbesondere aus C₂-C₁₀-Alkylen, substituiert oder vorzugsweise nicht substituiert, beispielsweise -CH₂-, -CH(CH₃)-, -CH(C₂H₅)-, -C(CH₃)₂-, -CH₂-CH₂-, -CH(CH₃)-CH₂-, -CH₂-CH(CH₃)-, -CH(C₂H₅)-CH₂-, -CH₂-CH(C₂H₅)-, -(CH₂)₃-, -(CH₂)₄-, -(CH₂)₅-, -(CH₂)₆-, -(CH₂)₈-, -(CH₂)₁₀-, bevorzugt -CH₂-CH₂-, -CH(CH₃)-CH₂-, -CH₂-CH(CH₃)-, ganz besonders bevorzugt -CH₂-CH₂-,
- n: verschieden oder gleich und gewählt aus null bis 200, bevorzugt 1 bis 150, besonders bevorzugt 5 bis 50,
- m: gewählt aus 1 bis 6, bevorzugt bis 4, besonders bevorzugt bis 2.

Dabei kann es sich bei der Variable n um einen Mittelwert handeln.

Bei Verbindung (B) kann es sich um ein Homopolymer handeln, das dann auch Polymer (B) genannt werden kann. Vorzugsweise handelt es sich jedoch um Copolymere, kurz Copolymere (B) genannt, gewählt aus Blockcopolymeren, Pfropfcopolymeren, alternierenden Copolymeren oder vorzugsweise aus statistischen Copolymeren. Verbindung (B) kann man beispielsweise durch ionische, beispielsweise anionische, oder vorzugsweise durch radikalische (Co)polymerisation von einem oder mehreren (Co)monomeren herstellen, von denen mindestens eines eine Struktureinheit der allgemeinen Formel I aufweist.

Struktureinheiten der Formel I können beispielsweise wie folgt aussehen:

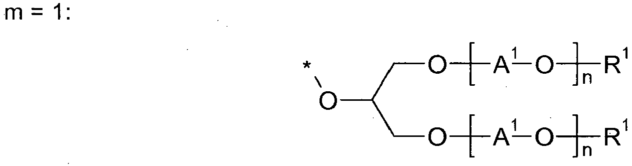

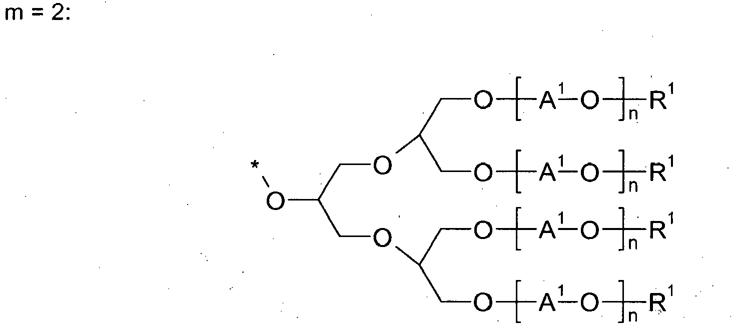

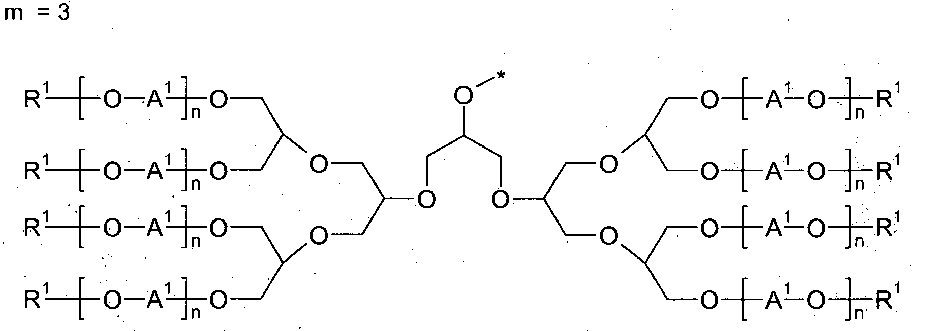

In einer Ausführungsform der vorliegenden Erfindung sind Verbindungen (B) verunreinigt mit Produkten einer unvollständige Umsetzung. Aufgrund einer unvollständigen Umsetzung können dann beispielsweise folgende Struktureinheiten gebildet werden:

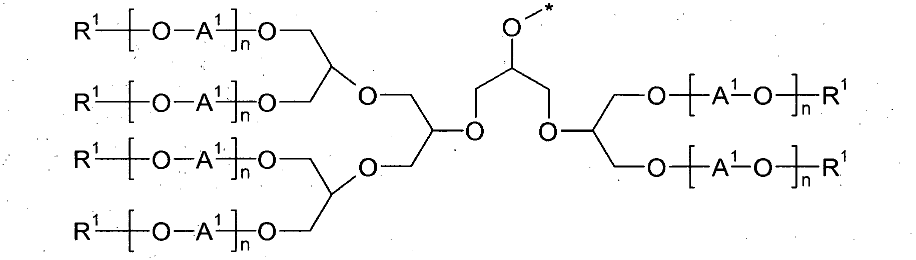

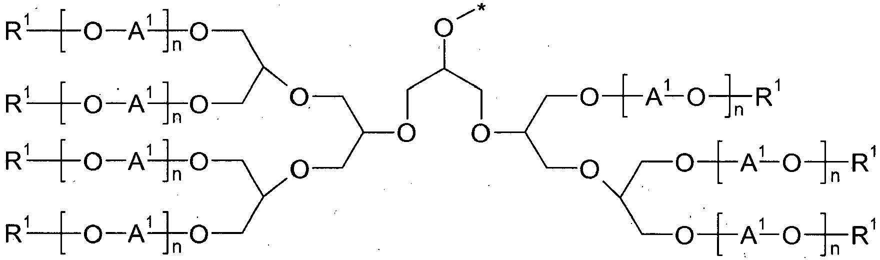

Für den Fall, dass man als Copolymer statistische Copolymere einzusetzen wünscht, kann man solche Copolymere einsetzen, die neben Struktureinheiten der allgemeinen Formel I noch mindestens eine Struktureinheit aufweisen, die auf das Comonomer zurückzuführen ist, wobei beliebige radikalisch polymerisierbare Comonomere geeignet sind, beispielsweise Vinylaromaten, insbesondere Styrol, Vinylester, insbesondere Vinylacetat, ethylenisch ungesättigte Carbonsäuren, insbesondere (Meth)acrylsäure, Amide oder Ester von ethylenisch ungesättigten Carbonsäuren, insbesondere C₁-C₁₀-Alkylester der (Meth)acrylsäure und (Meth)acrylnitril, weiterhin Maleinsäureanhydrid, halogenhaltige Comonomere wie beispielsweise Vinylchlorid oder Vinylidenchlorid und α-Olefine, beispielsweise 1-Decen, 1-Hexen, Isobuten oder 1-Dodecen.

Für den Fall, dass man als Copolymer statistische Copolymere einzusetzen wünscht, kann man solche Copolymere einsetzen, die neben Struktureinheiten der allgemeinen Formel I mindestens eine Struktureinheit der allgemeinen Formel II aufweisen, 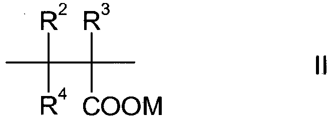 wobei die Variablen wie folgt definiert sind:
- R², R³: gleich oder verschieden und gewählt aus C₁-C₆-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl; bevorzugt n-C₁-C₄-Alkyl wie Methyl, Ethyl, n-Propyl, iso-Propyl, n-Butyl, iso-Butyl, sec.-Butyl und tert.-Butyl, besonders bevorzugt Methyl
und insbesondere Wasserstoff,
- R⁴: C₁-C₆-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl; bevorzugt n-C₁-C₄-Alkyl wie Methyl, Ethyl, n-Propyl, iso-Propyl, n-Butyl, iso-Butyl, sec.-Butyl und tert.-Butyl, besonders bevorzugt Methyl
und insbesondere COOM oder Wasserstoff,
- M: gewählt aus Ammonium, substituiert wie beispielsweise Methylammonium, Dimethylammonium, Trimethylammonium, Ethanolammonium, oder vorzugsweise unsubstituiert, und insbesondere Wasserstoff und Metallkationen, beispielsweise Alkalimetallkationen wie Li⁺, Na⁺, K⁺, Rb⁺ oder Cs⁺, bevorzugt Na⁺ oder K⁺. M kann auch für ein halbes Äquivalent eines Erdalkalimetallkations stehen, beispielsweise für ein halbes Äquivalent Ca²⁺ oder Mg²⁺.

In einer Ausführungsform der vorliegenden Erfindung ist R³ Methyl, und R² und R⁴ sind jeweils Wasserstoff.

In einer Ausführungsform der vorliegenden Erfindung sind R², R³ und R⁴ jeweils Wasserstoff.

In einer Ausführungsform der vorliegenden Erfindung sind R² und R³ jeweils Wasserstoff, und R⁴ ist COOM.

In einer Ausführungsform der vorliegenden Erfindung weist Verbindung (B) ein Molekulargewicht M_{w} im Bereich von 200 bis 100.000 g/mol auf, bevorzugt 1.000 bis 10.000 g/mol, besonders bevorzugt bis 5.000 g/mol, bestimmt beispielsweise durch Gelpermeationschromatograhie (GPC).

In einer Ausführungsform der vorliegenden Erfindung weist Verbindung (B) einen K-Wert nach Fikentscher im Bereich von 8 bis 40 auf, gemessen bei 23°C in THF/Wasser-Gemischen, vorzugsweise in 2 Gew.-% THF in Wasser.

In einer Ausführungsform der vorliegenden Erfindung weist Verbindung (B) eine Polydispersität M_{w}/Mₙ im Bereich von 1 bis 4 auf, bevorzugt von 1,1 bis 2 und besonders bevorzugt im Bereich von 1,1 bis 1,5.

In einer Ausführungsform der vorliegenden Erfindung weist Verbindung (B) eine Hydroxylzahl im Bereich von 1 bis 270, bevorzugt 5 bis 10, besonders bevorzugt 10 bis 70 mg KOH/g auf, bestimmt nach DIN 53240.

In einer Ausführungsform der vorliegenden Erfindung weist erfindungsgemäßes Copolymer ein Molekulargewicht M_{w} im Bereich von 1.000 bis 10.000.000 g/mol auf, bevorzugt 10.000 bis 1.000.000 g/mol, besonders bevorzugt 50.000 bis 500.000 g/mol, bestimmt beispielsweise durch Gelpermeationschromatograhie (GPC).

In einer Ausführungsform der vorliegenden Erfindung weist erfindungsgemäßes Copolymer eine Polydispersität M_{w}/Mₙ im Bereich von 1 bis 10 auf.

In einer Ausführungsform der vorliegenden Erfindung liegt das molare Verhältnis von Struktureinheiten der Formel I zu Struktureinheiten der Formel II in erfindungsgemäßem Copolymer im Bereich von 0,01 bis 10, bevorzugt 0,05 bis 2 und besonders bevorzugt 0,1 bis 0,5.

In einer Ausführungsform der vorliegenden Erfindung ist mindestens eine Struktureinheit der allgemeinen Formel I über eine Gruppe der Formel III a 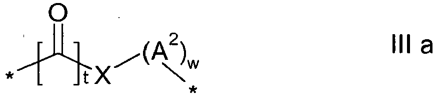 oder III b 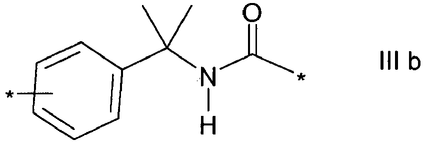 an das Grundgerüst von erfindungsgemäßem Copolymer gebunden, wobei die Variablen wie folgt gewählt werden:
- X: wird gewählt aus einer Einfachbindung, N-H und vorzugsweise Sauerstoff,
- t: wird gewählt aus null und eins,
- w: wird gewählt aus null und eins,
- A²: wird gewählt aus C₁-C₅₀-Alkylen, substituiert oder vorzugsweise nicht substituiert, wobei eine oder mehrere nicht-benachbarte CH₂-Gruppen durch Sauerstoff ersetzt sein können. Beispiele für geeignete Gruppen A² sind C₂-C₁₀-Alkylen, bevorzugt -CH₂-CH₂-, -CH(CH₃)-CH₂-, -CH₂-CH(CH₃)-, besonders bevorzugt -CH₂-CH₂-, weiterhin -CH₂-CH₂-O-, -CH(CH₃)-CH₂-O-, -CH₂-CH(CH₃)-O-, -[CH₂-CH₂-O-]_{y}-, -[CH(CH₃)-CH₂-O-]_{y}-, -[CH₂-CH(CH₃)-O-]_{y}-, wobei y eine ganze Zahl im Bereich von 2 bis 20, bevorzugt 2 bis 15 und insbesondere 3 bis 10 ist.

Erfindungsgemäße Copolymere enthalten weiterhin mindestens ein weiteres Comonomer (C) einpolymerisiert Geeignete Comonomere sind ethylenisch ungesättigte Verbindungen, die sich radikalisch mit den Comonomeren (A) und (B) copolymerisieren lassen. Beispielhaft sind zu nennen: C₁-C₁₀-Alkylester von ethylenisch ungesättigten Mono- oder Dicarbonsäuren, insbesondere von (Meth)acrylsäure, Vinylacetat, Vinylaromaten wie insbesondere Styrol und α-Methylstyrol, α-Olefine wie insbesondere C₁₂-C₂₀-α-Olefine, weiterhin Vinylchlorid, Acrylnitril und N-Vinylpyrrolidon. Bevorzugte Beispiele für C₁-C₁₀-Alkylester von ethylenisch ungesättigten Monocarbonsäuren sind Methyl(meth)acrylat, Ethyl(meth)acrylat, n-Butyl(meth)acrylat und 2-Ethylhexyl(meth)acrylat.

In einer Ausführungsform der vorliegenden Erfindung enthält erfindungsgemäßes Copolymer mindestens zwei verschiedene weitere Comonomere (C) einpolymerisiert. In einer anderen Ausführungsform der vorliegenden Erfindung enthält erfindungsgemäßes Copolymer genau ein weiteres Comonomer (C) einpolymerisiert.

In einer Ausführungsform der vorliegenden Erfindung enthält erfindungsgemäßes Copolymer einpolymerisiert:
0,1 bis 99 Gew.-%, bevorzugt 5 bis 70 Gew.-%, besonderes bevorzugt 10 bis 50 Gew.-% ethylenisch ungesättigte Mono- oder Dicarbonsäure (A),
0,001 bis 99 Gew.-%, bevorzugt 0,01 bis 50 Gew.-%, besonderes bevorzugt 0,1 bis 10 Gew.-% Verbindung (B),
insgesamt 0.1 bis 99 Gew.-%, bevorzugt 5 bis 80 Gew.-%, besonderes bevorzugt 30 bis 60 Gew.-% weitere(s) Comonomer(e) (C).

Dabei sind Angaben in Gew.-% jeweils auf gesamtes erfindungsgemäßes Copolymer bezogen.

Ein weiterer Gegenstand der vorliegenden Erfindung ist die Verwendung von erfindungsgemäßen Copolymeren als Verdicker, insbesondere als Assoziativ-Verdicker. Dabei kommt erfindungsgemäßen Copolymeren ihre Eigenschaft zu Gute, dass sie ein stark pH-Wert abhängiges Löslichkeitsverhalten in wässrigem Milieu aufweisen. Ein weiterer Aspekt der vorliegenden Erfindung ist demgemäß die Verwendung der erfindungsgemäßen Copolymerisate als Zusatz in Wasch- und Reinigungsmitteln sowie in kosmetischen Zubereitungen. Gegenstand ist weiterhin ein Verfahren zur Reinigung von textilen Substraten unter Verwendung der erfindungsgemäßen Copolymerisate.

Ein weiterer Gegenstand der vorliegenden Erfindung sind Wasch- und Reinigungsmittel sowie kosmetische Zubereitungen, enthaltend mindestens ein erfindungsgemäßes Copolymer. Dabei sind unter Waschmitteln hauptsächlich Waschmittel für Textil zu verstehen, während Reinigungsmittel harte Oberflächen wie beispielsweise Porzellan, Keramik, Glas, Stein, Holz oder Beton betreffen. Unter kosmetischen Zubereitungen sind nicht nur Salben, Lotions, Peelings und andere Zubereitungen, die sich unmittelbar als kosmetische Zubereitungen eignen, zu verstehen, sondern auch solche Zubereitungen, die als Basis für kosmetische Verbraucherprodukte verwendet werden.

Erfindungsgemäße Copolymere können dabei in Form der freien Säuren oder in partiell oder vollständig neutralisierter Form eingesetzt werden, beispielsweise mit Ammonium-, Erdalkali- oder vorzugsweise Alkalimetallkationen partiell oder vollständig neutralisiert. Bevorzugt setzt man erfindungsgemäße Copolymere in nicht neutralisierter Form, also als freie Säure, ein und neutralisiert erst unmittelbar vor oder während des Wäsch- oder Reinigungsvorgangs.

Erfindungsgemäße Wasch- und Reinigungsmittel bzw. erfindungsgemäße kosmetische Zubereitungen können in flüssiger oder fester Form vorliegen.

Erfindungsgemäße feste Waschmittel enthalten vorzugsweise folgende Komponenten:
(a) 0,05 bis 20 Gew.-% mindestens eines erfindungsgemäßen Copolymers,
(b) 0,5 bis 40 Gew.-% mindestens eines nichtionischen, anionischen und/oder kationischen Tensids,
(c) 0,5 bis 50 Gew.-% eines anorganischen Builders,
(d) 0 bis 10 Gew.-% eines organischen Cobuilders und
(e) 0 bis 60 Gew.-% anderer üblicher Inhaltsstoffe, wie Stellmittel, Enzyme, Parfum, Komplexbildner, Korrosionsinhibitoren, Bleichmittel, Bleichaktivatoren, Bleichkatalysatoren, Farbübertragungsinhibitoren, weitere Vergrauungsinhibitoren, Soil-Release-Polyester, Faser- und Farbschutzadditve, Silicone, Farbstoffe, Bakterizide, Auflösungsverbesserer und/oder Sprengmittel,
wobei die Summe der Komponenten (a) bis (e) vorzugsweise 100 Gew.-% ergibt.

Erfindungsgemäße feste Waschmittelformulierungen können in Pulver-, Granulat-, Extrudat- oder Tablettenform vorliegen.

Erfindungsgemäße flüssige Waschmittelformulierungen haben vorzugsweise folgende Zusammensetzung:
(a) 0,05 bis 20 Gew.-% mindestens eines erfindungsgemäßen Copolymers,
(b) 0,5 bis 40 Gew.-% mindestens eines nichtionischen, anionischen und/oder kationischen Tensids,
(c) 0 bis 20 Gew.-% eines anorganischen Builders,
(d) 0 bis 10 Gew.-% eines organischen Cobuilders,
(e) 0 bis 60 Gew.-% anderer üblicher Inhaltsstoffe, wie Soda, Enzyme, Parfum, Komplexbildner, Korrosionsinhibitoren, Bleichmittel, Bleichaktivatoren, Bleichkatalysatoren, Farbübertragungsinhibitoren, weitere Vergrauungsinhibitoren , Soil-Release-Polyester, Faser- und Farbschutzadditve, Silicone, Farbstoffe, Bakterizide, organische Lösungsmittel, Löslichkeitsvermittler, Hydrotrope, Verdicker und/oder Alkanolamine und
(f) 0 bis 99,45 Gew.-% Wasser.

Als nichtionische Tenside (b) eignen sich dabei vor allem:
- Alkoxylierte C₈-C₂₂-Alkohole, wie Fettalkoholalkoxylate, Oxoalkohotalkoxylate und Guerbetalkoholalkoxylate: Die Alkoxylierung kann mit C₂-C₂₀ -Alkylenoxiden, vorzugsweise mit Ethylenoxid, Propylenoxid und/oder Butylenoxid erfolgen. Es können Blockcopolymerisate oder statistische Copolymere vorliegen. Pro mol Alkohol enthalten sie üblicherweise 1 bis 50 mol, vorzugsweise 1 bis 20 mol, mindestens eines Alkylenoxids. Besonders bevorzugtes Alkylenoxid ist Ethylenoxid. Die Alkohole haben vorzugsweise 10 bis 18 Kohlenstoffatome.
- Alkylphenolalkoxylate, insbesondere Alkylphenolethoxylate, die C₆-C₁₄-Alkylketten und 5 bis 30 mol Alkylenoxid/mol enthalten.
- Alkylpolyglucoside, die C₈-C₂₂-, vorzugsweise C₁₀-C₁₈-Alkylketten und in der Regel 1 bis 20, vorzugsweise 1,1 bis 5 Glucosideinheiten enthalten.
- N-Alkylglucamide, Fettsäureamidalkoxylate, Fettsäurealkanolamidalkoxylate sowie Blockcopolymere aus Ethylenoxid, Propylenoxid und/oder Butylenoxid.

Geeignete anionische Tenside sind beispielsweise:
- Sulfate von (Fett)alkoholen mit 8 bis 22, vorzugsweise 10 bis 18 Kohlenstoffatomen, insbesondere C₉-C₁₁-Alkoholsulfate, C₁₂-C₁₄-Alkoholsulfate, C₁₂-C₁₈-Alkoholsulfate, Laurylsulfat, Cetylsulfat, Myristylsulfat, Palmitylsulfat, Stearylsulfat und Talgfettalkoholsulfat.
- Sulfatierte alkoxylierte C₈-C₂₂-Alkohole (Alkylethersulfate): Verbindungen dieser Art werden beispielsweise dadurch hergestellt, dass man zunächst einen C₈-C₂₂-, vorzugsweise einen C₁₀-C₁₈-Alkohol, z.B. einen Fettalkohol, alkoxyliert und das Alkoxylierungsprodukt anschließend sulfatiert. Für die Alkoxylierung verwendet man vorzugsweise Ethylenoxid.
- Lineare C₈-C₂₀-Alkylbenzolsulfonate (LAS), vorzugsweise lineare C₉-C₁₃-Alkylben-zolsulfonate und -Alkyltoluolsulfonate.
- Alkansulfonate, insbesondere C₈-C₂₄-, vorzugsweise C₁₀-C₁₈-Alkansulfonate.
- Seifen, wie die Na- und K-Salze von C₈-C₂₄-Carbonsäuren.

Anionisches Tensid wird dem Waschmittel vorzugsweise in Form von Salzen zugegeben. Geeignete Kationen sind dabei z.B. Alkalimetallionen, wie Natrium, Kalium und Lithium, und Ammoniumsalze, wie Hydroxyethylammonium-, Di(hydroxyethyl)-ammonium- und Tri(hydroxyethyl)ammoniumsalze.

Als besonders geeignete kationische Tenside seien genannt:
- C₇-C₂₅-Alkylamine;
- N,N-Dimethyl-N-(hydroxy-C₇-C₂₅-alkyl)ammoniumsalze;
- mit Alkylierungsmitteln quaternisierte Mono- und Di-(C₇-C₂₅-alkyl)dimethylammoniumverbindungen;
- Esterquats, insbesondere quaternäre veresterte Mono-, Di- und Trialkanolamine, die mit C₈-C₂₂-Carbonsäuren verestert sind;
- Imidazolinquats, insbesondere 1-Alkylimidazoliniumsalze der Formeln VI oder VII 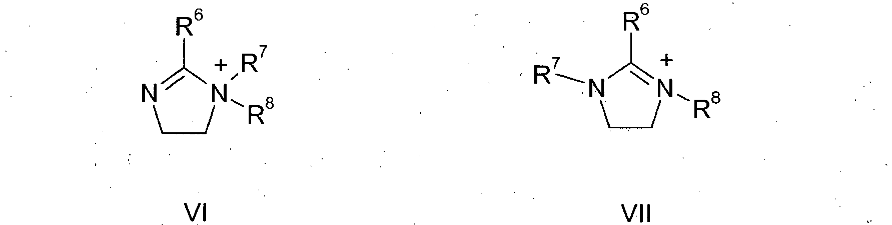
in denen die Variablen folgende Bedeutung haben:
- R⁶: gleich oder verschieden und gewählt aus C₁-C₂₅-Alkyl oder C₂-C₂₅-Alkenyl;
- R⁷: C₁-C₄-Alkyl oder Hydroxy-C₁-C₄-alkyl;
- R⁸: C₁-C₄-Alkyl, Hydroxy-C₁-C₄-alkyl oder ein Rest R⁶-(CO)-Y-(CH₂)ₚ- (Y Sauerstoff oder -NH-; p: 2 oder 3),
wobei für den Fall, dass R⁸ gleich R⁶-(CO)-Y-(CH₂)ₚ- ist, mindestens ein Rest R⁶ für C₇-C₂₂-Alkyl steht.

Als anorganische Builder eignen sich insbesondere:
- Kristalline und amorphe Alumosilikate mit ionenaustauschenden Eigenschaften, wie vor allem Zeolithe: Verschiedene Typen von Zeolithen sind geeignet, insbesondere die Zeolithe A, X, B, P, MAP und HS in ihrer Na-Form oder in Formen, in denen Na teilweise gegen andere Kationen wie Li, K, Ca, Mg oder Ammonium ausgetauscht ist,
- Kristalline Silikate, wie insbesondere Disilikate und Schichtsilikate, z.B. δ- und β-Na₂Si₂O₅. Die Silikate können in Form ihrer Alkalimetall-, Erdalkalimetall- oder Ammoniumsalze eingesetzt werden, bevorzugt sind die Na-, Li- und Mg-Silikate,
- Amorphe Silikate, wie Natriummetasilikat und amorphes Disilikat,
- Carbonate und Hydrogencarbonate: Diese können in Form ihrer Alkalimetall-, Erd-alkalimetall- oder Ammoniumsalze eingesetzt werden. Bevorzugt sind Na-, Li- und Mg-Carbonate und -Hydrogencarbonate, insbesondere Natriumcarbonat und/oder Natriumhydrogencarbonat,
- Polyphosphate, wie Pentanatriumtriphosphat.

Als organische Cobuilder eignen sich vor allem:
- Niedermolekulare Cärbonsäuren, wie Citronensäure, hydrophob modifizierte Citronensäure, z. B. Agaricinsäure, Äpfelsäure, Weinsäure, Gluconsäure, Glutarsäure, Bernsteinsäure, Imidodibernsteinsäure, Oxydibernsteinsäure, Propantricarbonsäure, Butantetracarbonsäure, Cyclopentantetracarbonsäure, Alkyl- und Alkenylbernsteinsäuren und Aminopolycarbonsäuren, z.B. Nitrilotriessigsäure, β-Alanin-diessigsäure, Ethylendiaminte traessigsäure, Serindiessigsäure, Isoserindiessigsäure, N-(2-Hydroxyethyl)iminodiessigsäure, Ethylendiamindibernsteinsäure und Methyl- und Ethylglycindiessigsäure.
- Oligomere und polymere Carbonsäuren, wie Homopolymere von Acrylsäure und Asparaginsäure, Oligomaleinsäuren, Copolymere der Maleinsäure mit Acrylsäure, Methacrylsäure oder C₂-C₂₂-Olefinen, z.B. Isobuten oder langkettigen α-Olefinen, mit Vinyl-C₁-C₈-alkylether, Vinylacetat, Vinylpropionat, (Meth)acrylsäureester von C₁-C₈-Alkoholen und/oder Styrol, Copolymere der Acrylsäure mit Methacrylsäure oder C₂-C₂₂-Olefinen, z.B. Isobuten oder langkettigen α-Olefinen, insbesondere mit C₁₀-C₂₂-α-Olefinen, Vinyl-C₁-C₈-alkylether, Vinylacetat, Vinylpropionat, (Meth)acrylsäureester von C₁-C₈-Alkoholen und Styrol. Bevorzugt sind die Homopolymere der Acrylsäure und Copolymere von Acrylsäure mit Maleinsäure. Oligomeren und polymeren Carbonsäuren werden in Säureform oder als Natriumsalz eingesetzt.

Geeignete Bleichmittel sind beispielsweise Addukte von Wasserstoffperoxid an anorganische Salze, wie Natriumperborat-Monohydrat, Natriumperborat-Tetrahydrat und Natriumcarbonat-Perhydrat, und Percarbonsäuren, wie Phthalimidopercapronsäure.

Als Bleichaktivatoren eignen sich z.B. N,N,N',N'-Tetraacetylethylendiamin (TAED), Natrium-p-nonanoyloxybenzolsulfonat und N-Methylmorpholiniumacetonitrilmethyl-sulfat.

Vorzugsweise in Waschmitteln eingesetzte Enzyme sind Proteasen, Lipasen, Amylasen, Cellulasen, Oxidasen und Peroxidasen.

Geeignete Farbübertragungsinhibitoren sind beispielsweise Homo-, Co- und Pfropfpolymere von 1-Vinylpyrrolidon, 1-Vinylimidazol oder 4-Vinylpyridin-N-oxid. Auch mit Chloressigsäure umgesetzte Homo- und Copolymere des 4-Vinylpyridins eignen sich als Farbübertragungsinhibitoren.

Weitere an sich übliche Waschmittelinhaltsstoffe sind bekannt. Detaillierte Beschreibungen sind z. B. in WO 99/06524 und WO 99/04313 und in "Liquid Detergents", Editor: Kuo-Yann Lai, Surfactant Sci. Ser., Vol. 67, Marcel Decker, New York, 1997, Seite 272-304, zu finden.

Es wurde außerdem gefunden, dass man erfindungsgemäße Copolymerisate und wässrige Dispersionen oder Lösungen von erfindungsgemäßem Copolymerisat als Verdicker in Reinigern für harte Oberflächen verwenden kann. Gegenstand der vorliegenden Erfindung ist daher die Verwendung von erfindungsgemäßen Copolymerisaten und von wässrigen Dispersionen oder Lösungen von erfindungsgemäßem Copolymerisat als Zusatzmittel für Reiniger für harte Oberflächen. Ein weiterer Gegenstand der vorliegenden Erfindung sind Reiniger für harte Oberflächen, enthaltend erfindungsgemäßes Copolymerisat. Ein weiterer Gegenstand der vorliegenden Erfindung ist ein Verfahren zur Reinigung von harten Oberflächen unter Verwendung von erfindungsgemäßem Copolymerisat oder von wässrigen Dispersionen oder Lösungen von erfindungsgemäßem Copolymerisat.

Unter Reinigern für harte Oberflächen sollen beispielsweise Reiniger für die Metall-, Kunststoff-, Glas- und Keramikreinigung, Fußbodenreiniger, Sanitärreiniger, Allzweckreiniger im Haushalt und in gewerblichen Anwendungen, technische Reiniger (für den Einsatz in Autowaschanlagen oder Hochdruckreinigern), Kaltreiniger, Geschirreiniger, Klarspüler, Desinfektionsreiniger, Reiniger für die Nahrungsmittel- und Getränkeindustrie, insbesondere als Flaschenreiniger, als CIP-Reiniger (Cleaning-in-Place) in Molkereien, Brauereien und sonstigen Betrieben von Nahrungsmittelherstellern verstanden werden. Reiniger, welche erfindungsgemäßes Copolymerisat enthalten, eignen sich besonders zum Reinigen harter Oberflächen wie Glas, Kunststoff und Metall. Die Reiniger können alkalisch, sauer oder neutral eingestellt sein. Sie enthalten üblicherweise ein oder mehrere Tenside in Mengen von etwa 0,2 bis 50 Gew.-%. Dabei kann es sich um anionische, nicht-ionische oder kationische Tenside sowie um Mischungen von Tensiden handeln, die miteinander verträglich sind, z.B. Mischungen aus anionischen und nicht-ionischen oder aus kationischen und nicht-ionischen Tensiden. Alkalische Reiniger können enthalten Soda, Pottasche, Natriumhydrogencarbonat, Kaliumhydrogencarbonat, Natriumsesquicarbonat, Kaliumsesquicarbonat, Natriumhydroxid, Kaliumhydroxid, Aminbasen wie Monoethanolamin, Diethanolamin, Triethanolamin, Ammoniak oder Silikat in Mengen bis zu 60 Gew.-%, in einigen Fällen sogar bis zu 80 Gew.-%. Erfindungsgemäße Reiniger für harte Oberflächen können außerdem Zitrate, Glukonate oder Tartrate in Mengen bis zu 80 Gew.-% enthalten. Erfindungsgemäße Reiniger für harte Oberflächen können in fester oder in flüssiger Form vorliegen.

In einer Ausführungsform der vorliegenden Erfindung ist erfindungsgemäßes Copolymerisat in erfindungsgemäßen Reinigern für harte Oberflächen in Mengen von 0,1 bis 20, vorzugsweise 0,2 bis 15 Gew.-% enthalten.

Ein weiterer Gegenstand der vorliegenden Erfindung sind kosmetische Zubereitungen, enthaltend mindestens ein erfindungsgemäßes Copolymer. Erfindungsgemäße kosmetische Zubereitungen können beispielsweise zur Pflege von Haut, Haar, Hornhaut, Finger- oder Fußnägeln oder zur Mundpflege eingesetzt werden.

Erfindungsgemäße kosmetische Zubereitungen können als wässrige oder wässrigalkoholische Lösungen, als O/W- oder W/O-Emulsionen vorliegen.

In einer Ausführungsform der vorliegenden Erfindung liegen erfindungsgemäße kosmetische Zubereitungen in Form von Shampoos, Cremes, Schäumen, Sprays (Pumpspray oder Aerosol), Gelen, Gelsprays, Lotionen oder Mousse liegen und können dementsprechend mit üblichen weiteren Hilfsstoffen formuliert werden.

Bei den erfindungsgemäßen kosmetischen Zubereitungen kann es sich um hautkosmetische, haarkosmetische, pharmazeutische, hygienische oder pharmazeutische Mittel handeln. Aufgrund ihrer filmbildenden Eigenschaften eignen sich die zuvor beschriebenen Copolymere insbesondere als Zusatzstoffe für Haar- und Hautkosmetika.

Vorzugsweise liegen erfindungsgemäße kosmetische Zubereitungen in Form eines Sprays, eines Gels, eines Schaums, einer Salbe, Creme, einer Emulsion, einer Süspension, einer Lotion, einer Milch oder einer Paste vor. Gewünschtenfalls können auch Liposomen oder Mikrosphären eingesetzt werden.

Neben erfindungsgemäßem Copolymer können erfindungsgemäße kosmetische Zubereitungen mindestens einen Träger enthalten. Beispiele für geeignete Träger sind:
i) Wasser,
ii) wassermischbare organische Lösungsmittel, vorzugsweise C₂-C₄-Alkanolen, insbesondere Ethanol,
iii) Öle, Fette, Wachse,
iv) von iii) verschiedenen Ester von C₆-C₃₀-Monocarbonsäuren mit ein-, zwei- oder dreiwertigen Alkoholen,
v) gesättigte acyclische und cyclische Kohlenwasserstoffen,
vi) Fettsäuren,
vii) Fettalkohole,
viii) Treibgase,
und Mischungen davon.

Beispiele für Träger sind insbesondere Öle und Fette, gewählt aus Kohlenwasserstoffen geringer Polarität, wie Mineralölen; linearen gesättigten Kohlenwasserstoffen, vorzugsweise mit mehr als 8 C-Atomen wie Tetradecan, Hexadecan, Octadecan etc., cyclischen Kohlenwasserstoffen wie Decahydronaphthalin, verzweigten Kohlenwasserstoffen; tierischen und pflanzlichen Ölen, Wachsen, Wachsestern, Vaselin, Estern, bevorzugt Estern von Fettsäuren, wie z. B. die Ester von C₁-C₄₀-Monoalkanolen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat, Salicylaten, wie C₁-C₁₀-Salicylaten, z. B. Octylsalicylat, Benzoatestern wie C₁₀-C₁₅-Alkylbenzoaten, Benzylbenzoat, anderen kosmetischen Estern, wie Fettsäuretriglyceriden, Propylenglykolmonolaurat, Polyethylenglykolmonolaurat, C₁₀-C₁₅-Alkyllactaten und Mischungen davon.

Als Träger geeignete Siliconöle sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Als Träger bevorzugte Öl- bzw. Fettkomponenten sind ausgewählt unter Paraffin und Paraffinölen, Vaselin, natürlichen Fetten und Ölen wie Castoröl, Sojaöl, Erdnussöl, Olivenöl, Sonnenblumenöl, Sesamöl, Avocadoöl, Kakaobutter, Mandelöl, Pfirsichkernöl, Ricinus-öl, Lebertran, Schweineschmalz, Walrat, Spermacetöl, Spermöl, Weizenkeimöl, Macadamianussöl, Nachtkerzenöl, Jojobaöl, Fettalkoholen, wie Laurylälkohol, Myristylalkohol, Cetylalkohol, Stearylalkohol, Oleylalkohol, Cetylalkohol, Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Neben erfindungsgemäßem Copolymer und Träger können erfindungsgemäße kosmetische Zubereitungen enthalten: wenigstens einen von Träger und erfindungsgemäßem Copolymer verschiedenen Bestandteil, der ausgewählt ist unter kosmetisch wirksamen Pflege- und Wirkstoffen wie AHA-Säuren, Fruchtsäuren, Ceramiden, Phytantriol, Collagen, Vitaminen und Provitaminen, beispielsweise Vitamin A, E und C, Retinol, Bisabolol, Parithenol, Emulgatoren und Co-Emulgatoren, Tensiden, Konservierungsmitteln, Parfümölen, Haarpolymeren, Haar- und Hautconditionern, Pfropfpolymeren, Silikonverbindungen, wasserlöslichen oder dispergierbaren silikonhaltigen Polymeren, natürlichen und synthetischen Lichtschutzmitteln, Bleichmitteln, Gelbildnern, Pflegemitteln, Färbemitteln, Farbstoffen, Pigmenten, Mikropigmente wie Titanoxid oder Zinkoxid, Trübungsmitteln, Tönungsmitteln, Bräunungsmitteln, Konsistenzgebern, Feuchthaltemitteln, Rückfettern, Collagen, Eiweißhydrolysaten, Lipiden, Antioxidantien, Entschäumern, Antistatika, Emollienzien, Lösungsvermittlern, Repellents, Überfettungsmitteln, Perlglanzwachsen, Konsistenzgebern, Solubilisatoren, Komplexbildnern, pH-Wert Regulatoren, Reflektoren, Proteinen und Proteinhydrolysaten (z.B. Weizen-, Mandel- oder Erbsenproteine) und Weichmachern.

Ein weiterer Gegenstand der vorliegenden Erfindung sind Verbindungen (B). Erfindungsgemäße Verbindungen (B) weisen pro Molekül mindestens eine Struktureinheit der allgemeinen Formel I auf,

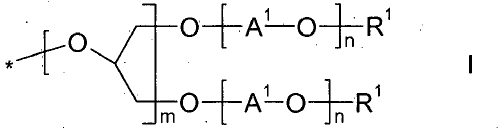

Dabei sind die Variablen wie vorstehend definiert:
- R¹: verschieden oder vorzugsweise gleich und gewählt aus Wasserstoff oder bevorzugt C₆-C₃₀-Aryl, bevorzugt C₆-C₁₄-Aryl, insbesondere Phenyl; C₇-C₃₀-Aralkyl, bevorzugt Benzyl; C₃-C₁₀-Cycloalkyl, insbesondere Cyclopentyl oder Cyclohexyl, und insbesondere C₁-C₃₀-Alkyl, bevorzugt C₁-C₂₀-Alkyl.
- A¹: verschieden oder vorzugsweise gleich und gewählt aus C₆-C₁₀-Arylen, vorzugsweise Phenylen, C₇-C₁₀-Aralkylen wie beispielsweise -CH(C₆H₅)- oder -CH₂-CH(C₆H₅)-, und insbesondere aus C₂-C₁₀-Alkylen, substituiert oder vorzugsweise nicht substituiert, bevorzugt -CH₂-CH₂-, -CH(CH₃)-CH₂-, -CH₂-CH(CH3)-, ganz besonders bevorzugt -CH₂-CH₂-,
- n: verschieden oder gleich und gewählt aus null bis 200, bevorzugt 1 bis 150, besonders bevorzugt 5 bis 50,
- m: gewählt aus 1 bis 6, bevorzugt bis 4, besonders bevorzugt bis 2.

In einer Ausführungsform der vorliegenden Erfindung weist erfindungsgemäße Verbindung (B) ein Molekulargewicht M_{w} im Bereich von 200 bis 100.000 g/mol auf, bevorzugt bis 10.000 g/mol, besonders bevorzugt bis 5.000 g/mol, bestimmt beispielsweise durch Gelpermeationschromatograhie (GPC). Ein geeigneter Standard ist beispielsweise Polymethylmethacrylat (PMMA).

In einer Ausführungsform der vorliegenden Erfindung weist Verbindung (B) einen K-Wert nach Fikentscher im Bereich von 8 bis 40 auf, gemessen bei 23°C in THF/Wasser-Gemischen, vorzugsweise in 2 Gew.-% Lösung in THF.

In einer Ausführungsform der vorliegenden Erfindung weist erfindungsgemäßes Verbindung (B) eine Polydispersität Mₙ/M_{w} im Bereich von 1 bis 4 auf, bevorzugt von 1,1 bis 2 und besonders bevorzugt im Bereich von 1,1 bis 1,5.

In einer Ausführungsform der vorliegenden Erfindung ist mindestens eine Struktureinheit der allgemeinen Formel I über eine Gruppe der Formel III a 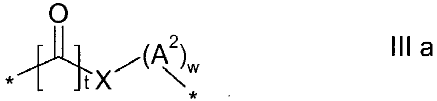 oder III b 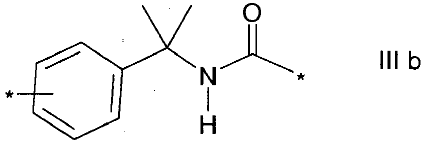 an das Grundgerüst von erfindungsgemäßem Copolymer gebunden.

In einer Ausführungsform der vorliegenden Erfindung enthält erfindungsgemäße Verbindung (B) mindestens ein Comonomer der allgemeinen Formel IV a oder IV b einpolymerisiert, 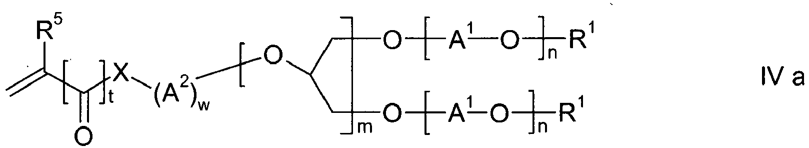 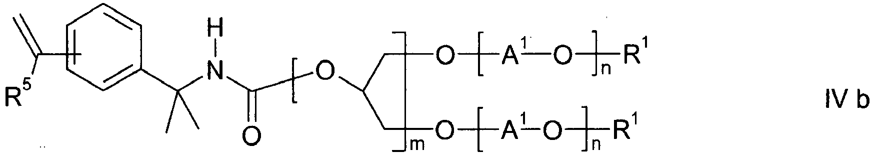 wobei R⁵ gewählt ist aus Methyl und Wasserstoff und die übrigen Variablen wie vorstehend definiert sind.

Ein weiterer Gegenstand der vorliegenden Erfindung ist ein Verfahren zur Herstellung von erfindungsgemäßen Verbindungen (B), das dadurch gekennzeichnet ist, dass man mindestens ein (Co)monomer der allgemeinen Formel IV a oder IV b 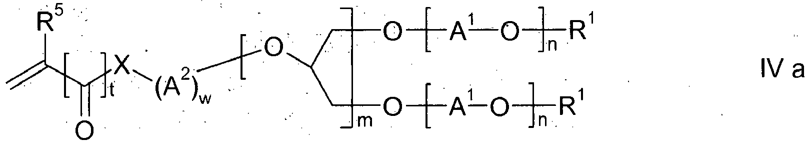 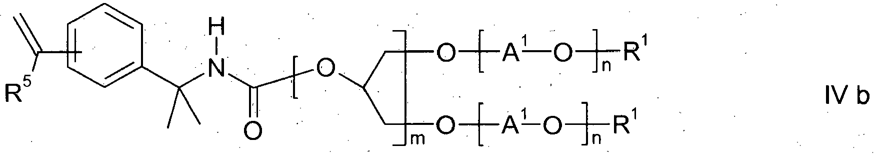 vorzugsweise radikalisch polymerisiert, vorzugsweise in Gegenwart von mindestens einem Comonomer der allgemeinen Formel V a bis V c 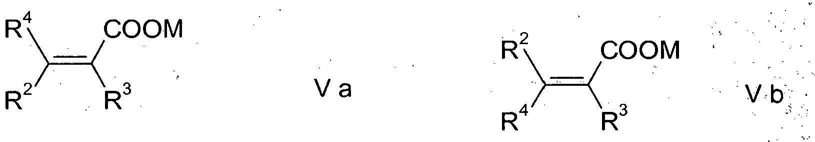 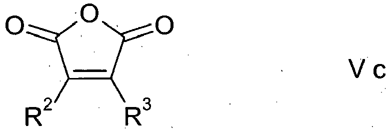 wobei die Variablen wie folgt definiert sind:
- R², R³: gleich oder verschieden und gewählt aus C₁-C₆-Alkyl, bevorzugt n-C₁-C₄-Alkyl, besonders bevorzugt Methyl und insbesondere Wasserstoff,
- R⁴: C₁-C₆-Alkyl, bevorzugt n-C₁-C₄-Alkyl, besonders bevorzugt Methyl, bevorzugt COOM und insbesondere Wasserstoff,
- M: gewählt aus Ammonium, substituiert oder vorzugsweise unsubstituiert, Wasserstoff und Metallkationen, insbesondere Alkalimetallkationen oder einem halben Äquivalent Erdalkalimetallkationen,
- R⁵: gewählt aus Wasserstoff und Methyl,
- t: gewählt aus null und eins.
- w: gewählt aus null und eins,
- A²: wird gewählt aus C₁-C₅₀-Alkylen, substituiert oder vorzugsweise nicht substituiert, wobei eine oder mehrere nicht-benachbarte CH₂-Gruppen durch Sauerstoff ersetzt sein können. Beispiele für geeignete Gruppen A² sind C₂-C₁₀-Alkylen, bevorzugt -CH₂-CH₂-, -CH(CH₃)-CH₂-, -CH₂-CH(CH₃)-, besonders bevorzugt -CH₂-CH₂-, weiterhin -CH₂-CH₂-O-, -CH(CH₃)-CH₂-O-, -CH₂-CH(CH₃)-O-, -[CH₂-CH₂-O]_{y}-, -[CH(CH₃)-CH₂-O]_{y}-, -[CH₂-CH(CH₃)-O-]_{y}, wobei y eine ganze Zahl im Bereich von 2 bis 20, bevorzugt 2 bis 15 und insbesondere 3 bis 10 ist.

Beispielsweise kann man erfindungsgemäße Verbindungen (B) wie folgt herstellern.

Man stellt zunächst eine Verbindung der allgemeinen Formel VI a her, 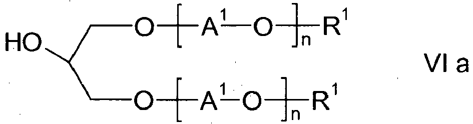 in der die Variablen wie vorstehend beschrieben sind und in der m = 1 gewählt ist. Dazu setzt man mindestens eine Verbindung der allgemeinen Formel R¹-(O-A¹)ₙ-OH mit Glycerin oder vorzugsweise einem reaktiven Derivat von Glycerin um, insbesondere mit Epichlorhydrin. Dazu wählt man vorzugsweise ein Molverhältnis von Verbindung der allgemeinen Formel R¹-(O-A¹)ₙ-OH zu reaktivem Derivat von Glycerin, insbesondere Epichlorhydrin, wie 2 : 1. Wünscht man Verbindungen (B) herzustellen, die Struktureinheiten der allgemeinen Formel I aufweisen, aber bei denen m > 1, so setzt man anschließend weiteres Glycerin oder weiteres reaktives Derivat von Glycerin ein. Man erhält solche Verbindungen der allgemeinen Formel VI, 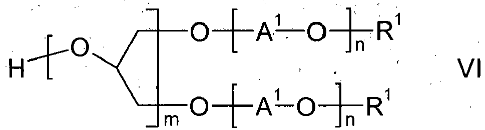 bei denen m > 1 ist. Die Herstellung von Verbindung der allgemeinen Formel VI bzw. VI a kann man beispielsweise bei Temperaturen im Bereich von 100 bis 110°C durchführen. Die Herstellung von Verbindung der allgemeinen Formel VI a kann man auch bei Temperaturen unter 100°C durchführen. Je höher der Wert von m in Verbindung der allgemeinen Formel VI gewünscht wird, desto höhere Temperaturen sind vorteilhaft, beispielsweise bis zu 120°C.

Die Herstellung von Verbindung der allgemeinen Formel VI bzw. VI a kann man in Gegenwart eines Katalysators durchführen. Geeignete Katalysatoren sind beispielsweise anorganische und organische Basen. Setzt man Epichlorhydrin als reaktives Glycerinderivat ein, so dient Base nicht nur als Katalysator, sondern auch zum Neutralisieren der entstehenden HCl. Geeignete anorganische Basen sind beispielsweise Alkalimetallcarbonate und insbesondere Alkalimetallhydroxide wie NaOH und KOH. Geeignete organische Basen sind beispielsweise tertiäre Amine, insbesondere Triethylamin und [2,2,2]Diazabicyclooctan (DABCO), sowie Pyridin und para-N,N-Dimethylaminopyridin.

In einer Ausführungsform der vorliegenden Erfindung kann man die Herstellung von Verbindung der allgemeinen Formel VI bzw. VIa in einem Lösungsmittel durchführen. Geeignete Lösungsmittel sind beispielsweise Ether, insbesondere 1,4-Dioxan, Diisopropylether, Tetrahydrofuran ("THF") und Di-n-butylether. Weitere geeignete Lösungsmittel sind n-Butylacetat ("Butylacetat"), DMSO, N,N-Dimethylformamid ("DMF") und N-Methylpyrrolidon und aromatische Lösungsmittel wie beispielsweise Toluol.

In Ausführungsformen, in denen bei der Herstellung von Verbindung der allgemeinen Formel VI Wasser abgespalten wird, kann man Wasser entziehendes Mittel einsetzen, beispielsweise Molekularsieb, Natriumsulfat, Magnesiumsulfat, oder man kann das gebildete Wasser durch azeotrope Destillation entfernen.

In einer Ausführungsform der vorliegenden Erfindung führt man die Umsetzung zu Verbindung der allgemeinen Formel VI über einen Zeitraum von 15 Minuten bis 48 Stunden durch, bevorzugt 1 bis 24 Stunden, besonderes bevorzugt 3 bis 15 Stunden.

In einer Ausführungsform der vorliegenden Erfindung führt man die Umsetzung zu Verbindung der allgemeinen Formel VI stufenweise durch, und zwar in so vielen Stufen, wie sie dem gewünschten m entspricht. Dabei wird reaktives Derivat von Glycerin, insbesondere Epichlorhydrin in der betreffenden Zahl an Stufen zugesetzt. Zur stufeweisen Umsetzung kann man beispielsweise so vorgehen, dass man zunächst eine bestimmte Menge an Verbindung der allgemeinen Formel R¹-(O-A¹)ₙ-OH mit der halben Molzahl an Glycerin oder vorzugsweise mit einem reaktiven Derivat von Glycerin, insbesondere mit Epichlorhydrin, umsetzt. Danach gibt man eine Menge an Glycerin oder an reaktivem Derivat von Glycerin zu, die einem Viertel der Molzahl von Verbindung der allgemeinen Formel R¹-(O-A¹)ₙ-OH entspricht, und setzt um. Wünscht man eine weitere Stufe durchzuführen, so gibt man danach eine Menge an Glycerin oder an reaktivem Derivat von Glycerin zu, die einem Achtel der Molzahl an Verbindung der allgemeinen Formel R¹-(O-A¹)ₙ-OH entspricht, und setzt um. Bei jeder weiteren Stufe reduziert man die zugesetzte Molzahl an Verbindung der allgemeinen Formel R¹-(O-A¹)ₙ-OH entsprechend.

In einem weiteren Schritt kann man Verbindung der allgemeinen Formel VI mit einer Verbindung mit einer ethylenischen Doppelbindung umsetzen.

Beispielsweise kann man Verbindung der allgemeinen Formel VI mit einem hinreichend reaktiven Derivat einer ethylenisch ungesättigten Carbonsäure oder einer ethylenisch ungesättigten Dicarbonsäure, beispielsweise mit Säurechlorid oder Säureanhydrid, insbesondere mit (Meth)acrylsäureanhydrid umsetzen und erhält erfindungsgemäße Verbindung der Formel IV a, in der t = 1 ist und die übrigen Variablen wie vorstehend definiert sind.

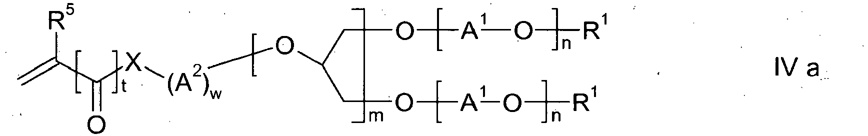

In einer Ausführungsform der vorliegenden Erfindung führt man die Umsetzung von Verbindung der allgemeinen Formel VI zu Verbindung der allgemeinen Formel IV bei Temperaturen im Bereich von - 20 bis +70 °C, bevorzugt von +15 bis 50 °C durch.

In einer Ausführungsform der vorliegenden Erfindung führt man die Umsetzung von Verbindung der allgemeinen Formel VI zu Verbindung der allgemeinen Formel IV in einem Lösungsmittel oder einem Gemisch von Lösungsmitteln durch. Geeignete Lösungsmittel sind beispielsweise Ether, insbesondere Tetrahydrofuran, Diethylether, 1,4-Dioxan, Aceton, oder apolare Lösungsmittel wie beispielsweise N,N-Dimethylformamid ("DMF"), Dimethylsulfoxid (DMSO) oder Kohlenwasserstoffe, beispielsweise Cyclohexan.

In einer Ausführungsform der vorliegenden Erfindung führt man die Umsetzung von Verbindung der allgemeinen Formel VI zu Verbindung der allgemeinen Formel IV in Gegenwart von Katalysator durch. Geeignete Katalysatoren sind beispielsweise Basen, beispielsweise Natriumhydrid und insbesondere tertiäre Amine wie beispielsweise N,N-Dimethylaminopyridin. Katalysatoren werden im Allgemeinen in unterstöchiometrischen Mengen eingesetzt. Wünscht man jedoch, die als reaktives Säurederivat ein Säurehalogenid einzusetzen, so kann man mit einem Überschuss an Base arbeiten, wobei die Base gleichzeitig die gebildete Säure neutralisiert und als Katalysator dienen kann.

In einer Ausführungsform der vorliegenden Erfindung führt man die Umsetzung von Verbindung der allgemeinen Formel VI zu Verbindung der allgemeinen Formel IV über einen Zeitraum von 15 Minuten bis 12 Stunden durch, bevorzugt sind 1 bis 5 Stunden.

In einer anderen Ausführungsform der vorliegenden Erfindung kann man Verbindung der allgemeinen Formel VI mit Acetylen umsetzen und erhält erfindungsgemäße Verbindung der Formel IV a, in der t = null ist und die übrigen Variablen wie vorstehend definiert sind.

Zur Umsetzung mit Acetylen kann man einen oder mehrere Katalysatoren, bevorzugt gewählt aus basischen Katalysatoren einsetzen. Besonders geeignet ist KOH.

Die Umsetzung mit Acetylen kann man mit oder ohne Lösungsmittel durchführen. Geeignete Lösungsmittel sind beispielsweise N-Methylpyrrolidon, N-Ethylpyrrolidon, Toluol, Xylol, THF und Dioxan.

Die Umsetzung mit Acetylen kann man beispielsweise bei Temperaturen im Bereich von 80 bis 160°C durchführen, bevorzugt sind Temperaturen um 120°C, beispielsweise 110 bis 130°C.

Die Umsetzung mit Acetylen kann man bei Normaldruck durchführen oder vorzugsweise bei erhöhtem Druck, beispielsweise bei 2 bis 30 bar.

In einer Ausführungsform der vorliegenden Erfindung führt man die Umsetzung mit Acetylen über einen Zeitraum von 15 Minuten bis 48 Stunden durch, bevorzugt bis 36 Stunden.

In einer anderen Ausführungsform der vorliegenden Erfindung kann man Verbindung der allgemeinen Formel VI mit einem Diisocyanat, beispielsweise TMXDI (Tetramethylxylylendiisocyanat) umsetzen und erhält erfindungsgemäße Verbindung der allgemeinen Formel IV b, in der die Variablen wie vorstehend definiert sind.

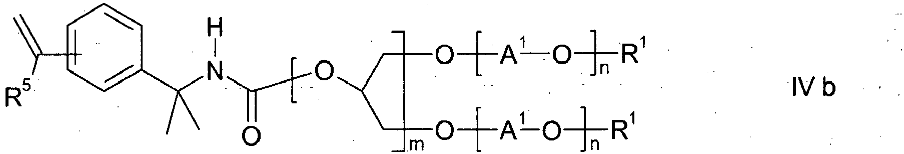

In einer Ausführungsform der vorliegenden Erfindung kann man die Herstellung von erfindungsgemäßen Verbindungen der Formel IV b in Gegenwart eines Katalysators durchführen, beispielsweise in Gegenwart einer Zinn-organischen Verbindung, insbesondere In Gegenwart von DBTL (Di-n-butylzinndilaurat).

In einer Ausführungsform der vorliegenden Erfindung kann man die Herstellung von erfindungsgemäßen Verbindungen der Formel IV b in Gegenwart eines Lösungsmittels durchführen. Geeignet sind beispielsweise cyclische und nichtcyclische Ether, beispielsweise THF, 1,4-Dioxan und Di-n-butylether, weiterhin Aceton und unpolare Lösungsmittel, beispielsweise Cyclohexan.

In einer Ausführungsform der vorliegenden Erfindung kann man die Herstellung von erfindungsgemäßen Verbindungen der Formel IV b bei einer Temperatur im Bereich von 20 bis 100 °C, bevorzugt 20 bis 80 °C durchführen.

In einer Ausführungsform der vorliegenden Erfindung kann man die Herstellung von erfindungsgemäßen Verbindungen der Formel IV b über eine Reaktionsdauer im Bereich von 1 bis 10 Stunden, bevorzugt 2 bis 5 Stunden durchführen.

Wünscht man solche erfindungsgemäße Verbindungen herzustellen, bei denen R¹ Wasserstoff ist, so ist es bevorzugt, zunächst als R¹ eine Schutzgruppe einzuführen, beispielsweise eine Acetatschutzgruppe, die man in einem späteren Reaktionsschritt abspaltet, beispielsweise unter sauren wässrigen Bedingungen.

Andere bevorzugte Schutzgruppen sind Acetal- und Ketalschutzgruppen, beispielsweise

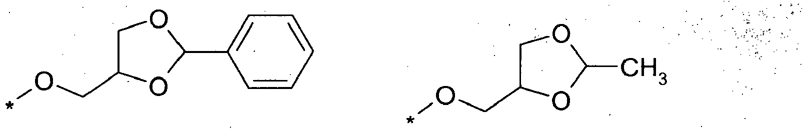

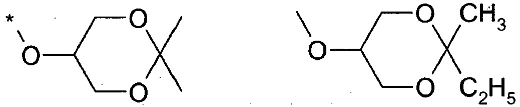

Wünscht man auf Basis einer Acetal- oder Ketalschutzgruppe eine erfindungsgemäße Verbindung der allgemeinen Formel IV aufzubauen, so geht man vorzugsweise von einem acetalisierten oder ketalisierten Triol aus, beispielsweise von acetalisiertem oder ketalisiertem Glycerin oder acetalisiertem oder ketalisiertem Trimethylolpropan. Man setzt unter den vorstehend beschriebenen Bedingungen einmal oder mehrfach mit Epichlorhydrin um und erhält ein verzweigtes Molekül, das man mit beispielsweise Säurechlorid oder Säureanhydrid oder einem anderen geeigneten Derivat einer ethylenisch ungesättigten Carbonsäure zu Verbindung der allgemeinen Formel IV a oder mit einem geeigneten Diisocyanat zu Verbindung der allgemeinen Formel IV b umsetzen kann. Durch Hydrolyse unter schwach sauren Bedingungen kann man die Acetalgruppe bzw. Ketalgruppe abspalten, wenn es gewünscht ist, und erhält erfindungsgemäße Verbindungen mit R¹ = Wasserstoff.

Besonders bevorzugte Ketal-Schutzgruppe ist die Isopropyliden-Schutzgruppe.

Natürlich ist es möglich, nach jedem der vorstehend genannten Arbeitsschritte eine oder mehrere Reinigungsoperationen durchzuführen. So ist es beispielsweise bevorzugt, entstandene Halogenide abzutrennen, beispielsweise durch Filtration. Weiterhin ist es bevorzugt, bei vermindertem Druck zu trocknen, beispielsweise um flüchtige Verunreinigungen abzutrennen.

Ein weiterer Gegenstand der vorläufigen Erfindung ist ein Verfahren zur Herstellung von erfindungsgemäßen Copolymeren.

Die erfindungsgemäße Herstellung von erfindungsgemäßen Copolymeren kann man beispielsweise so durchführen, dass man mit einem geeigneten Initiator eine vorzugsweise radikalische Copolymerisation von Verbindung der allgemeinen Formel IV a und/oder IV b sowie Comonomer (A) und Comonomer(en) (C) auslöst, gegebenenfalls in Gegenwart von mindestens einem Comonomer der allgemeinen Formel V a bis-V c, 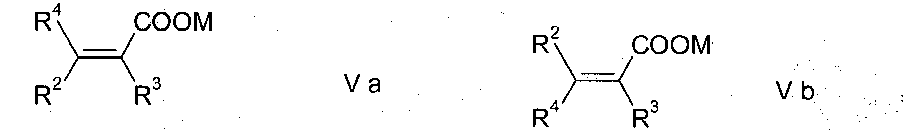 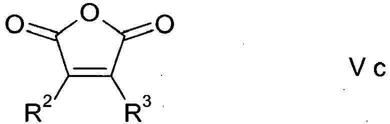 wobei die Variablen wie folgt definiert sind:
- R², R³: gleich oder verschieden und gewählt aus C₁-C₆-Alkyl, bevorzugt n-C₁-C₄-Alkyl, besonders bevorzugt Methyl und insbesondere Wasserstoff,
- R⁴: C₁-C₆-Alkyl, bevorzugt n-C₁-C₄-Alkyl, besonders bevorzugt Methyl, bevorzugt COOM und insbesondere Wasserstoff,
- M: gewählt aus Ammonium, substituiert oder vorzugsweise unsubstituiert, Wasserstoff und Metallkationen, insbesondere Alkalimetallkationen oder einem halben Äquivalent Erdalkalimetallkationen.

Geeignete Initiatoren sind insbesondere Radikalstarter, beispielsweise organische Peroxide, insbesondere organische Peroxide mit mindestens einer tert.-Butylgruppe oder mindestens einer tert.-Amylgruppe, und Azoverbindungen, beispielsweise Azobisisobutyronitril (AIBN). Auch Redoxinitiatoren sind geeignet, beispielsweise Kombinbationen aus Wasserstoffperoxid oder Natriumperoxodisulfat oder einem der vorstehend genannten Peroxide mit einem Reduktionsmittel. Als Reduktionsmittel sind beispielsweise geeignet: Ascorbinsäure, Weinsäure, Natriumbisulfit, Kaliumbisulfit, Fe(II)-Salze wie beispielsweise FeSO₄ oder Alkalimetallsalze von Chelaten von Fe(II). Weiterhin sind anorganische Peroxide geeignet, beispielsweise Kaliumperoxodisulfat und Natriumperoxodisulfat.

Die erfindungsgemäße Herstellung von erfindungsgemäßen Copolymeren durch Copolymerisation von Verbindung der allgemeinen Formel IV a und/oder IV b, im Rahmen der vorliegenden Erfindung auch erfindungsgemäße Copolymerisation genannt, kann man beispielsweise unter Verwendung von mindestens einem Lösungsmittel durchführen. Geeignete Lösungsmittel können beispielsweise sein: N,N-Dimethylformamid (DMF), Dioxan, Toluol oder unpolare Lösungsmittel wie beispielsweise Cyclohexan.

In einer Ausführungsform der vorliegenden Erfindung ist Wasser ein geeignetes Lösungsmittel. Dies ist insbesondere dann der Fall, wenn man die erfindungsgemäße Copolymerisation mit anorganischem Peroxid zu starten wünscht.

In einer anderen Ausführungsform der vorliegenden Erfindung führt man die erfindungsgemäße Copolymerisation ohne Verwendung eines Lösungsmittels durch.

In einer Ausführungsform der vorliegenden Erfindung wählt man als Konzentration des bzw. der Comonomeren zu Beginn der erfindungsgemäßen Copolymerisation einen Bereich von 60 bis 95 Gew.-%, bevorzugt 70 bis 80 Gew.-% an Comonomer, bezogen auf sämtliche Comonomere. Man kann die erfindungsgemäße Copolymerisation auch als Massepolymerisation, also ohne Zugabe von Lösungsmittel, durchführen.

In einer Ausführungsform der vorliegenden Erfindung führt man die erfindungsgemäße Copolymerisation als Emulsionspolymerisation durch.

Die erfindungsgemäße Copolymerisation kann man als kontinuierliches Verfahren, als semikontinuierliches Verfahren oder in Form einer Batch-Copolymerisation durchführen.

In einer Ausführungsform der vorliegenden Erfindung führt man die erfindungsgemäße (Co)polymerisation bei Temperaturen im Bereich von 60 bis 90°C durch.

Durch die Ausübung des erfindungsgemäßen Verfahrens erhält man erfindungsgemäßes Copolymer.

Ein weiterer Gegenstand der vorliegenden Erfindung sind Verbindungen der allgemeinen Formel IV a 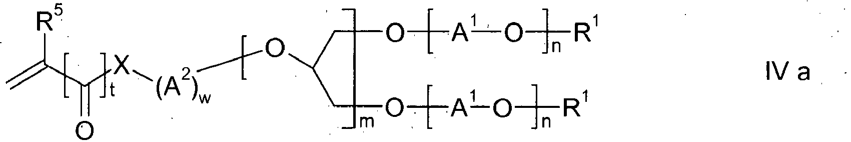 in denen die Variablen wie vorstehend definiert sind:
- R¹: verschieden oder vorzugsweise gleich und gewählt aus Wasserstoff oder bevorzugt C₆-C₃₀-Aryl, bevorzugt C₆-C₁₄-Aryl, insbesondere Phenyl; C₇-C₃₀-Aralkyl, bevorzugt Benzyl; C₃-C₁₀-Cycloalkyl, bevorzugt Cyclopentyl oder Cyclohexyl, und insbesondere C₁-C₃₀-Alkyl, bevorzugt C₁-C₂₀-Alkyl.
- A¹: verschieden oder vorzugsweise gleich und gewählt aus C₆-C₁₀-Arylen, vorzugsweise Phenylen, C₇-C₁₀-Aralkylen wie beispielsweise -CH(C₆H₅)- oder -CH₂-CH(C₆H₅)-, und insbesondere aus C₂-C₁₀-Alkylen, substituiert oder vorzugsweise nicht substituiert, bevorzugt -CH₂-CH₂-, -CH(CH₃)-CH₂-, -CH₂-CH(CH₃)-, ganz besonders bevorzugt -CH₂-CH₂-,
- n: verschieden oder gleich und gewählt aus null bis 200, bevorzugt 1 bis 150, besonders bevorzugt 5 bis 50,
- m: gewählt aus 1 bis 6, bevorzugt bis 4, besonders bevorzugt bis 2,
- R⁵: gewählt aus Wasserstoff und Methyl,
- t: gewählt aus null und eins.
- w: gewählt aus null und eins,
- A²: gewählt aus C₁-C₅₀-Alkylen, substituiert oder vorzugsweise nicht substituiert, wobei eine oder mehrere nicht-benachbarte CH₂-Gruppen durch Sauerstoff ersetzt sein können. Beispiele für besonders geeignete Gruppen A² sind C₂-C₁₀-Alkylen, bevorzugt -CH₂-CH₂-, -CH(CH₃)-CH₂-, -CH₂-CH(CH₃)-, besonders bevorzugt -CH₂-CH₂-, weiterhin -CH₂-CH₂-O-, -CH(CH₃)-CH₂-O-, -CH₂-CH(CH₃)-O-, -[CH₂-CH₂-O]_{y}-, -[CH(CH₃)-CH₂-O]_{y}-, -[CH₂-CH(CH₃)-O-]_{y}, wobei y eine ganze Zahl im Bereich von 2 bis 20, bevorzugt 2 bis 15 und insbesondere 3 bis 10 ist.

Ein weiterer Gegenstand der vorliegenden Erfindung sind Verbindungen der allgemeinen Formel IV b

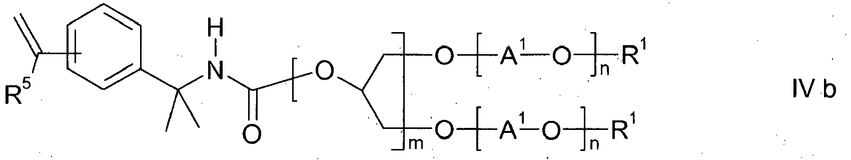

Dabei sind die Variablen wie vorstehend definiert:
- R¹: verschieden oder vorzugsweise gleich und gewählt aus Wasserstoff oder bevorzugt C₆-C₃₀-Aryl, bevorzugt C₆-C₁₄-Aryl, insbesondere Phenyl; C₇-C₃₀-Aralkyl, bevorzugt Benzyl; C₃-C₁₀-Cycloalkyl, bevorzugt Cyclopentyl oder Cyclohexyl, und insbesondere C₁-C₃₀-Alkyl, bevorzugt C₁-C₂₀-Alkyl;
- A¹: verschieden oder vorzugsweise gleich und gewählt aus C₆-C₁₀-Arylen, vorzugsweise Phenylen, C₇-C₁₀-Aralkylen wie beispielsweise -CH(C₆H₅)- oder -CH₂-CH(C₆H₅)-, und insbesondere aus C₂-C₁₀-Alkylen, substituiert oder vorzugsweise nicht substituiert, bevorzugt -CH₂-CH₂-, -CH(CH₃)-CH₂-, -CH₂-CH(CH₃)-, ganz besonders bevorzugt -CH₂-CH₂-,
- n: verschieden oder gleich und gewählt aus null bis 200, bevorzugt 1 bis 150, besonders bevorzugt 5 bis 50,
- m: gewählt aus 1 bis 6, bevorzugt bis 5, besonders bevorzugt 2 bis 4,
- R⁵: gewählt aus Wasserstoff und Methyl.

Erfindungsgemäße Verbindungen der allgemeinen Formel IV a und IV b sind beispielsweise sehr gut geeignet, um erfindungsgemäße Verbindungen (B) herzustellen. Ihre Herstellung gelingt beispielsweise nach dem vorstehend beschriebenen Verfahren.

Die Erfindung wird durch Arbeitsbeispiele erläutert.

Allgemeine Vorbemerkungen: Die Darstellung der Verbindungen der Formel VI und IV erfolgte in Lösungsmitteln, die nach Standardmethoden getrocknet waren.
Die Bestimmung der Molekulargewichte erfolgte durch GPC in DMAC (N,N-Dimethylacetamid als Lösemittel), Standard: PMMA.
Unter Dioxan wird stets 1,4-Dioxan verstanden, wenn nicht anders angegeben.
Die (Co)polymerisationen wurden unter Schutzgasatmosphäre durchgeführt (getrockneter Stickstoff).
Angaben in % sind stets Gewichts-%, wenn nicht ausdrücklich anders angegeben.

### I. Herstellung von Verbindungen der allgemeinen Formel VI

### 1.1 Herstellung von erfindungsgemäßer Verbindung VI.1

In einem 2-Liter-Kolben mit Tropftrichter, Magnetrührer und Rückflusskühler wurde eine Lösung von 400 g (0,29 mol) Polyethylenglykolmonostearylether, CH₃-(CH₂)₁₇-O-(CH₂CH₂-O)₂₅H, in 1150 ml Dioxan vorgelegt. Unter Rühren gab man 25 g KOH-Plätzchen zu. Man erwärmte auf 105°C und gab dann 14 g (0,15 mol) Epichlorhydrin, gelöst in 50 ml Dioxan, über einen Zeitraum von 15 Minuten tropfenweise zu. Man rührte die inzwischen rotbraune Mischung über einen Zeitraum von 15 Stunden bei 105°C und kühlte dann auf Zimmertemperatur ab. Man filtrierte den entstandenen Niederschlag ab und destillierte das Dioxan bei 30 mbar ab. Man erhielt erfindungsgemäße Verbindung VI.1, ein zähes hellbraunes Öl, das durch MS (Maldi TOF) und durch GPC charakterisiert wurde. Mₙ: 3200 g/mol, M_{w}: 4200 g/mol.

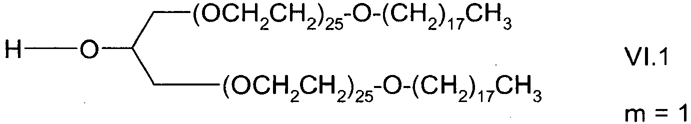

### I.2 Herstellung von erfindungsgemäßer Verbindung VI.2

In einem 2-Liter-Kolben mit Tropftrichter, Magnetrührer und Rückflusskühler wurde eine Lösung von 400 g (0,53 mol) Polyethylenglykol-monomethylether (M_{w} = 750 g/mol, n = 16) in 1150 ml Dioxan vorgelegt. Unter Rühren gab man 30 g KOH-Plätzchen zu. Man erwärmte auf 105°C und gab dann 25 g (0,26 mol) Epichlorhydrin, gelöst in 50 ml Dioxan, über einen Zeitraum von 30 Minuten tropfenweise zu. Man rührte die so erhältliche Mischung über einen Zeitraum von 15 Stunden bei 105°C und kühlte dann auf Zimmertemperatur ab. Man filtrierte den entstandenen Niederschlag ab und destillierte das Dioxan bei 30 mbar ab. Man erhielt erfindungsgemäße Verbindung VI.2, ein zähes gelbliches Öl, das durch MS (Maldi TOF) und durch GPC charakterisiert wurde. Mₙ = 1800 g/mol, M_{w} = 2400 g/mol.

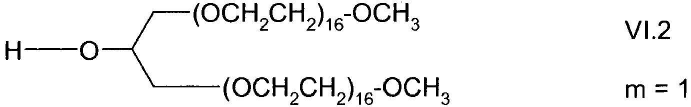

### I.3 Herstellung von erfindungsgemäßer Verbindung VI.3

In einem 2-Liter-Kolben mit Tropftrichter, Magnetrührer und Rückflusskühler wurde eine Lösung von 365 g (1,04 mol) H-(OCH₂CH₂)₇-O-n-C₁₂H₂₅ (M_{w} = 490 g/mol, n = 7) in 1600 ml Dioxan vorgelegt. Unter Rühren gab man 67 g KOH-Plätzchen zu. Man erwärmte auf 105°C und gab dann 35 g (0,37 mol) Epichlorhydrin, gelöst in 100 ml Dioxan, über einen Zeitraum von 30 Minuten tropfenweise zu. Man rührte die so erhältliche Mischung über einen Zeitraum von 15 Stunden bei 105°C und kühlte dann auf Zimmertemperatur ab. Man filtrierte den entstandenen Niederschlag ab und destillierte das Dioxan bei 30 mbar ab. Man erhielt erfindungsgemäße Verbindung VI.3, ein gelbliches Öl, das durch MS (Maldi TOF) und durch GPC charakterisiert wurde. Mₙ = 1470 g/mol, M_{w} = 1940 g/mol.

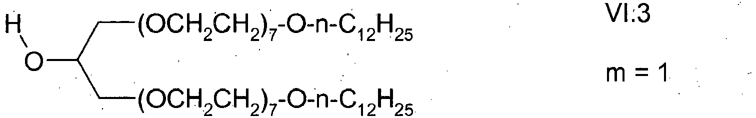

### I.4 Herstellung von erfindungsgemäßer Verbindung VI.4

In einem 4-Liter-Kolben mit Tropftrichter, Magnetrührer und Rückflusskühler wurde eine Lösung von 700 g (0,93 mol) H-(OCH₂CH₂)₁₆-OCH₃ (M_{w} = 750 g/mol, n = 16) in 1600 ml Dioxan vorgelegt. Unter Rühren gab man 224 g KOH-Plätzchen zu. Man erwärmte auf 105°C und gab dann 43 g (0,45 mol) Epichlorhydrin, gelöst in 175 ml Dioxan, über einen Zeitraum von 30 Minuten tropfenweise zu. Man rührte die so erhältliche Mischung über einen Zeitraum von 2 Stunden und gab dann tropfenweise weitere 21 g (0,23 mol) Epichlorhydrin in 100 ml Dioxan über einen Zeitraum von 30 Minuten zu. Man rührte die so erhältliche Mischung über einen Zeitraum von 2 Stunden und gab dann tropfenweise weitere 10,8 g (0,115 mol) Epichlorhydrin in 50 ml Dioxan über einen Zeitraum von 15 Minuten zu. Man rührte 15 Stunden bei 105°C und kühlte dann auf Zimmertemperatur ab. Man filtrierte den entstandenen Niederschlag ab und destillierte das Dioxan bei 30 mbar ab. Man erhielt erfindungsgemäße Verbindung VI.4, ein gelbliches Öl, das durch MS (Maldi TOF) und durch GPC charakterisiert wurde. Mₙ = 2400 g/mol, M_{w} = 3100 g/mol.

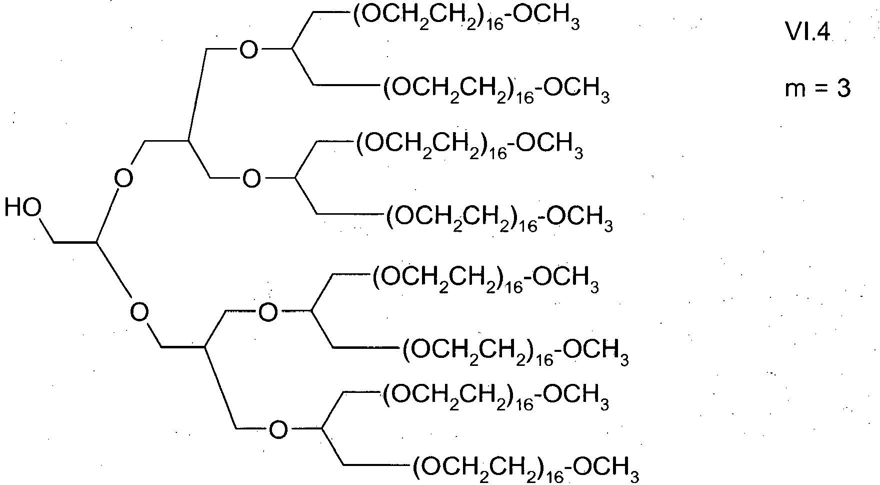

### II. Herstellung von erfindungsgemäßen Verbindungen der allgemeinen Formel IV

### 11.1 Herstellung von erfindungsgemäßer Verbindung IV.1 aus erfindungsgemäßer Verbindung VI.1

In einem 2000-ml-Kolben, der mit Tropftrichter und Magnetrührer ausgestattet war, wurde eine Lösung von 200 g Verbindung VI.1 in 800 ml THF vorgelegt. Man rührte 20 Minuten bei Zimmertemperatur und gab 50 mg para-N,N-Dimethylaminopyridin zu. Danach gab man über einen Zeitraum von 15 Minuten eine Lösung von 15,5 g Methacrylsäureanhydrid, gelöst in 50 ml THF, tropfenweise zu. Man beobachtete die Bildung einer geringen Menge an Niederschlag. Man rührte noch 3 Stunden bei Zimmertemperatur, trennte den Niederschlag durch Filtration ab und destillierte dann das THF bei Zimmertemperatur und verringertem Druck ab. Man erhielt erfindungsgemäße Verbindung IV.1 als braunes Öl, welche durch MALDI-TOF und GPC charakterisiert wurde.

### II.2 Herstellung von erfindungsgemäßer Verbindung IV.2 aus erfindungsgemäßer Verbindung VI.2

In einem 2-I-Kolben, der mit Tropftrichter und Magnetrührer ausgestattet war, wurde eine Lösung von 200 g Verbindung VI.2 in 800 ml THF vorgelegt. Man rührte 20 Minuten bei Zimmertemperatur und gab 50 mg para-N,N-Dimethylaminopyridin zu. Danach gab man über einen Zeitraum von 15 Minuten eine Lösung von 9 g Methacrylsäureanhydrid, gelöst in 50 ml THF, tropfenweise zu. Man beobachtete die Bildung einer geringen Menge an Niederschlag. Man rührte noch 3 Stunden bei Zimmertemperatur, trennte den Niederschlag durch Filtration ab und destillierte dann das THF bei Zimmertemperatur und verringertem Druck ab. Man erhielt erfindungsgemäße Verbindung IV.2 als braunen Feststoff.

### II.3 Herstellung von erfindungsgemäßer Verbindung IV.3 aus erfindungsgemäßer Verbindung VI.3

In einem 1-I-Kolben, der mit Tropftrichter und Magnetrührer ausgestattet war, wurde eine Lösung von 200 g Verbindung VI.3 in 600 ml THF vorgelegt. Man rührte 20 Minuten bei Zimmertemperatur und gab 50 mg para-N,N-Dimethylaminopyridin zu. Danach gab man über einen Zeitraum von 20 Minuten eine Lösung von 19 g Methacrylsäureanhydrid, gelöst in 50 ml THF, tropfenweise zu. Man rührte noch 3 Stunden bei Zimmertemperatur, filtrierte und destillierte dann das THF bei Zimmertemperatur und verringertem Druck ab. Man erhielt erfindungsgemäße Verbindung IV.3, die durch MALDI-TOF charakterisiert wurde.

### II.4 Herstellung von erfindungsgemäßer Verbindung IV.4 aus erfindungsgemäßer Verbindung VI.4

In einem 2,5-I-Autoklaven wurden 1000 g erfindungsgemäße Verbindung VI.4 und 10 g KOH vorgelegt, mit Stickstoff (2 bar) intertisiert und danach auf 120°C erhitzt. Danach wurde Acetylen mit einem Druck von 20 bar aufgepresst und die Reaktionsmischung bei 120°C und 20 bar gerührt, bis insgesamt 85 l Acetylen aufgenommen waren. Danach wurde auf Raumtemperatur abgekühlt, entspannt und der Rückstand nach Erwärmen auf 60°C für 3 h unter Rühren entgast und dann dem Autoklav entnommen. Verbindung IV.4 wurde durch ¹H-NMR-Spektroskopie charakterisiert.

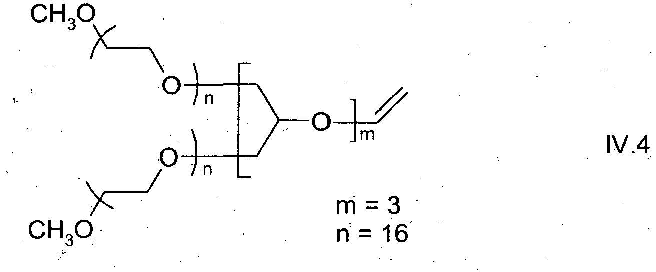

### III. Herstellung von erfindungsgemäßen Copolymeren

### Allgemeine Bemerkungen:

Aus denen erfindungsgemäßen Verbindungen IV.1 bis IV.3 wurden dispergierte erfindungsgemäße Copolymere hergestellt. Für die Anwendung dieser dispergierten erfindungsgemäßen Copolymere in Wasch- und Reinigungsmitteln sowie für Kosmetikartikel wird die Erfindung durch die folgenden Anwendungsbeispiele näher veranschaulicht.

### III.1 Herstellung eines erfindungsgemäßen Copolymers (CP.1)

Man stellte Emulsion E.3.1 her, bestehend aus
220 g vollständig entsalztes Wasser (VE-Wasser)
137,3 g Methacrylsäure (A.1)
155,3 g Ethylacrylat (C.1)
164,4 g n-Butylacrylat (C.2)
12,5 g erfindungsgemäße Verbindung IV.1 (B.1)
12,32 g einer 28 Gew.-% wässrigen Lösung von Laurylethersulfat als Emulgator E-mul.1.
8,63 g einer 0,4% wässrigen Ascorbinsäure-Lösung

In einer Rührapparatur, bestehend aus einem 2-Liter-HWS-Gefäß mit Ankerrührer (175 Upm), Rückflusskühler, Innenthermofühler und Dosierstation, wurden als Vorlage 675,9 g entsalztes Wasser (VE-Wasser), 0,2 g einer 4 Gew.-% [FeK₂(EDTA)]-Lösung (EDTA = Ethylendiamintetraacetat) und 12,3 g einer 28 Gew.-% wässrigen Lösung von Laurylethersulfat) miteinander vermischt. Man erwärmte auf 75°C.

Danach gab man 9,2 g einer 1 Gew.-% wässrigen Wasserstoffperoxid-Lösung zu. Danach wurden unter weiterem Rühren bei 75 °C 50% der Emulsion E.3.1 zudosiert. Anschließend wurde die Reaktionsmischung auf 65°C abgekühlt. Bei 65°C wurde übrige Emulsion E.3.1 zudosiert. Man rührte bei 65°C über einen Zeitraum von 23 Minuten. Danach wurden 6,6 g einer 7 Gew.-% wässrigen Na₂S₂O₈-Lösung und 86,2 g einer 0,4%igen Ascorbinsäure-Lösung zudosiert. Danach wurde die Reaktionsmischung auf Raumtemperatur abgekühlt. Man erhielt eine wässrige Dispersion von erfindungsgemäßem Copolymer CP.1, die 31 % Feststoffgehalt aufwies.

### iii.2 Herstellung der erfindungsgemäßen Copolymere CP.2 bis CP.10

Die Daten zu den erfindungsgemäßen Copolymeren CP.2 bis CP.10 finden sich in Tabelle 1.
Zur Herstellung von CP.2 bis CP.10 wurde wie unter III.1 vorgegangen, jedoch wurden die Anteile an Comonomeren variiert. Außerdem wurde Art und Menge an Emulgator gemäß Tabelle 1 eingesetzt.
Unter Emulgator Emul.2 ist Sulfobernsteinsäure-di-2-ethylhexylester-Na-Salz zu verstehen.
Die Mengenangaben für die Einsatzstoffe sind in Gewichtsteilen pro 100 reaktive Monomerteile (parts per hundred monomers; pphm) angegeben. Zur Charakterisierung der Dispersion wurden folgende Werte gemessen:
Feststoffgehalt, "Feststoff": Die betreffende Dispersion wurde 30 min bei 140 °C getrocknet und der Feststoffgehalt in Prozent aus dem Verhältnis Trockenrückstand zu Einwaage bestimmt.

Teilchendurchmesser: Die betreffende Dispersion wurde mit Wasser auf 0,01 % verdünnt und die Teilchendurchmesser über Lichtstreuung im "High Performance Particle Sizer 5001" (HPPS) von der Firma Malvern Instruments gemessen.

LD-Wert: Die betreffende Dispersion wurde mit Wasser auf 0,01 % verdünnt und die Lichtdurchlässigkeit (LD) der Dispersion im Vergleich zu reinem Wasser als Maß für den Teilchendurchmesser optisch im Hach DR/2010 gemessen.

**Tabelle 1: Synthese, Zusammensetzung und Eigenschaften von erfindungsgemäßen Copolymeren CP.1 bis CP.7**

| | (A.1) | (B) | (C.1) | (C.2) | Emulgator | Feststoff | Ø | LD-Wert |
|---|---|---|---|---|---|---|---|---|
| | jeweils [pphm] | | | | | [Gew.-%] | [nm] | |
| CP.1 | 29,25 | 1,25 (IV.1) | 35,75 | 33,75 | 1,5 (Emul.1) | 30,6 | 60 | 98 |
| CP.2 | 29,25 | 0,75 (IV.2) | 36,06 | 33,94 | 1,5 (Emul.1) | 30,7 | 95 | 98 |
| CP.3 | 28,2 | 2,5 (IV.1) | 34,67 | 34,67 | 3,0 (Emul.2) | 31,3 | 82 | 97 |
| CP.4 | 29,25 | 1,25 (IV.1) | 35,74 | 33,75 | 3,0 (Emul.2) | 30,7 | 68 | 95 |
| CP.5 | 29,25 | 1,25 (IV.1) | 35,74 | 33,75 | 1,5 (Emul.2) | 31,1 | 93 | 95 |
| CP.6 | 28,8 | 1,25 (IV.2) | 35,0 | 35,0 | 3,0 (Emul.2) | 31,2 | 84 | 96 |
| CP.7 | 30,0 | 2,5 (IV.1) | 33,75 | 33,75 | - | 30,3 | 64 | 98 |

### IV. Prüfung von erfindungsgemäßen Copolymeren in Flüssigwaschmitteln

Zur Anwendungsprüfung wurden erfindungsgemäße Copolymere in Stammformulierungen für Flüssigwaschmittel gemäß Tabelle 2 eingerührt und die verdickende Wirkung getestet. Die Stammformulierungen wurden aus den folgenden Stammformulierungen ausgewählt.

### Stammformulierung 1:

Man vermischte miteinander:
13,0 g para-n-C₁₀-C₁₃-Alkyl-C₆H₄-SO₃H,
7,5 g CH₃(CH₂)₁₁-(O-CH₂CH₂)₇-OH
8,5 g Kokosfettsäure (Fettsäuregemisch)
4,4 g KOH
3,0 g Natriumcitrat-dihydrat
8,0 g 1,2-Propylenglykol
2,0 g Ethanol
und füllte mit Wasser auf 90 g auf.

### Stammformulierung 2:

Man vermischte miteinander:
17,9 g para-n-C₁₀-C₁₃-Alkyl-C₆H₄-SO₃H
20,0 g CH₃(CH₂)₁₁-(O-CH₂CH₂)₇-OH
8,5 g Kokosfettsäure (Fettsäuregemisch)
5,0 g KOH
3,0 g Natriumcitrat-dihydrat
8,0 g 1,2-Propylenglykol
2,0 g Ethanol
und füllte mit Wasser auf 90 g auf.

### Stammformulierung 3:

Man vermischte miteinander:
13,4 g para-n-C₁₀-C₁₃-Alkyl-C₆H₄-S0₃H
10,0 g CH₃(CH₂)₁₁-O-CH₂CH₂)₇OH
8,5 g Kokosfettsäure (Fettsäuregemisch)
4,4 g KOH
3,0 g Natriumcitrat-dihydrat
8,0 g 1,2-Propylenglykol
2,0 g Ethanol
und füllte mit Wasser auf 90 g auf.

### Allgemeines Vorgehen:

Man vermischte 90 g Stammformulierung, wässrige Dispersion aus erfindungsgemäßem Copolymer und Wasser, so dass erfindungsgemäße Flüssigwaschmittel PWM.1 bis FWM.7 gemäß Tabelle 2 resultierten. Man ließ die erfindungsgemäßen Flüssigwaschmittel mindestens 5 Stunden bei Raumtemperatur ruhen und testete dann die anwendungstechnischen Eigenschaften. Die Resultate sind in Tabelle 2 zusammengefasst.

Als Referenzformulierung wurde jeweils die betreffende Stammformulierung mit Wasser auf 100 g verdünnt.

**Tabelle 2: Anwendungstechnische Prüfung von erfindungsgemäßen Flüssigwaschmitteln**

| FWM | Feststoff [%] | Stammformulierung | CP [g/100 g) | Viskosität [mPa·s] |
|---|---|---|---|---|
| V-FWM.0 | 31,0 | Nr. 1 | - | 78 |
| FWM.1 | 30,6 | Nr. 1 | 0,8 (CP.1) | 540 |
| FWM.2 | 30,7 | Nr. 1 | 0,8 (CP.2) | 570 |
| FWM.3 | 31,3 | Nr. 1 | 0,8 (CP.3) | 567 |
| FWM.4 | 30,7 | Nr. 1 | 0,8 (CP.4) | 633 |
| FWM.5 | 31,1 | Nr. 1 | 0,8 (CP.5) | 579 |
| FWM.6 | 31,2 | Nr. 1 | 0,8 (CP.6) | 564 |
| FWM.7 | 31,3 | Nr. 1 | 1,4 (CP.4) | 874 |
| FWM.8 | 30,7 | Nr. 1 | 1,4 (CP.5) | 1123 |
| FWM.9 | 31,1 | Nr. 1 | 1,4 (CP.6) | 1083 |
| FWM.10 | 30,3 | Nr. 2 | 1,5 (CP.7) | 927 |
| V-FWM.11 | 31,1 | Nr. 2 | - | 76 |
| FWM.12 | 30,3 | Nr. 3 | 1,5 (CP.7) | 902 |
| V-FWM.13 | 29,9 | Nr. 3 | - | 72 |

Die Viskosität wurde unter Berücksichtigung der Vorschriften nach DIN 51550, DIN 53018, DIN 53019 mit dem Brookfield Viskosimeter Modell RV-03 bei einer Drehzahl von 20 Umdrehungen pro Minute mit der Spindel Nr. 62 bei 20 °C gemessen. Die Viskosität der unverdickten Referenzformulierungen ist in Tab. 1 den Vergleichsbeispiel 1 bis 3 zu entnehmen.

Zur Quantifizierung der Transparenz der erfindungsgemäßen Flüssigwaschmittel wurde die Transmission in % bei 440 nm bei 23 °C mit einem LICO 200 der Fa. Dr. Lange gemessen. Die Transmission der unverdickten Referenzformulierung und der erfindungsgemäßen Flüssigwaschmittel lagen im gleichen Bereich zwischen 97 und 100%. Durch Zugabe von erfindungsgemäßen Copolymeren wurde im erfindungsgemäßen Flüssigwaschmittel keine Trübung verursacht.

## Patentansprüche

1. Copolymer, enthaltend als Comonomer einpolymerisiert
(A) mindestens eine ethylenisch ungesättigte Mono- oder Dicarbonsäure,
(B) mindestens eine ethylenisch ungesättigte Verbindung, die pro Molekül mindestens eine Struktureinheit der allgemeinen Formel I aufweist: 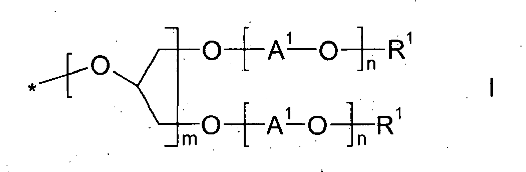 wobei die Variablen wie folgt definiert sind:
R¹ verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
A¹ verschieden oder gleich und gewählt aus C₂-C₁₀-Alkylen; C₆-C₁₀-Arylen und C₇-C₁₀-Aralkylen,
n verschieden oder gleich und gewählt aus null bis 200,
m verschieden oder gleich und gewählt aus 1 bis 6,
(C) mindestens ein weiteres Comonomer.

2. Copolymer nach Anspruch 1, **dadurch gekennzeichnet, dass** es mindestens eine Struktureinheit der allgemeinen Formel II aufweist, 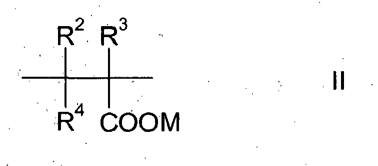 wobei die Variablen wie folgt definiert sind:
R², R³ gleich oder verschieden und gewählt aus C₁-C₆-Alkyl und Wasserstoff,
R⁴ C₁-C₆-Alkyl, COOM oder Wasserstoff,
M gewählt aus Ammonium, substituiert oder unsubstituiert, Wasserstoff und Metallkationen.

3. Copolymer nach Anspruch 1 oder 2, **dadurch gekennzeichnet, dass** Verbindung (B) ein Molekulargewicht M_{w} im Bereich von 200 bis 100.000 g/mol aufweist.

4. Copolymer nach einem der Ansprüche 1 bis 3, **dadurch gekennzeichnet, dass** Verbindung (B) einen K-Wert nach Fikentscher im Bereich von 8 bis 40 aufweist, gemessen in Wasser/THF-Gemischen.

5. Copolymer nach einem der Ansprüche 1 bis 4, **dadurch gekennzeichnet, dass** das molare Verhältnis von Struktureinheiten der Formel I zu Struktureinheiten der Formel II im Bereich von 0,01 bis 10 liegt.

6. Copolymer nach einem der Ansprüche 1 bis 5, **dadurch gekennzeichnet, dass** mindestens eine Struktureinheit der allgemeinen Formel I über eine Gruppe der Formel III a 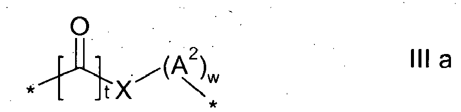 oder III b 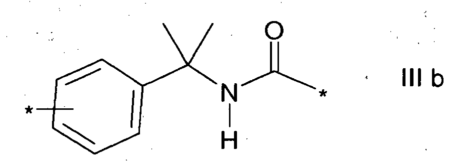 an das Grundgerüst von besagtem Copolymer gebunden ist, wobei die Variablen wie folgt definiert sind:
X wird gewählt aus einer Einfachbindung, Sauerstoff und N-H,
t wird gewählt aus null und eins,
w wird gewählt aus null und eins,
A² wird gewählt aus C₁-C₅₀-Alkylen, substituiert oder nicht substituiert, wobei eine oder mehrere nicht-benachbarte CH₂-Gruppen durch Sauerstoff ersetzt sein können.

7. Verwendung von Copolymeren nach einem der Ansprüche 1 bis 6 als Verdicker.

8. Wasch- oder Reinigungsmittel oder kosmetische Zubereitungen, enthaltend mindesten ein Copolymer nach einem der Ansprüche 1 bis 6.

9. Verbindungen (B), **dadurch gekennzeichnet, dass** sie pro Molekül mindestens eine Struktureinheit der allgemeinen Formel I-aufweisen: 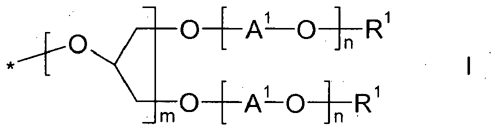 wobei die Variablen wie folgt definiert sind:
R¹ verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
A¹ verschieden oder gleich und gewählt aus C₂-C₁₀-Alkylen, C₆-C₁₀-Arylen und C₇-C₁₀-Aralkylen,
n verschieden oder gleich und gewählt aus null bis 200,
m verschieden oder gleich und gewählt aus 1 bis 6.

10. Verbindungen (B) nach Anspruch 9, **dadurch gekennzeichnet, dass** sie ein Molekulargewicht M_{w} im Bereich von 200 bis 100.000 g/mol aufweisen.

11. Verbindungen (B) nach Anspruch 9 oder 10, **dadurch gekennzeichnet, dass** sie mindestens ein Comonomer der allgemeinen Formel IV a oder IV b einpolymerisiert enthalten, 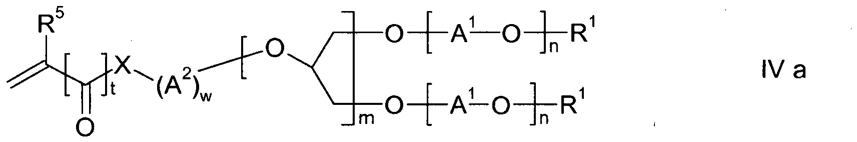 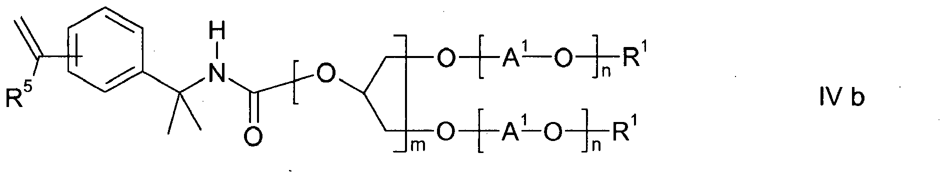 wobei die Variablen wie folgt definiert sind:
R¹ verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
A¹ verschieden oder gleich und gewählt aus C₂-C₁₀-Alkylen, C₆-C₁₀-Arylen und C₇-C₁₀-Aralkylen,
n verschieden oder gleich und gewählt aus null bis 200,
m verschieden oder gleich und gewählt aus 1 bis 6,
R⁵ gewählt aus Wasserstoff und Methyl,
t wird gewählt aus null und eins,
w wird gewählt aus null und eins,
X wird gewählt aus einer Einfachbindung, Sauerstoff und N-H,
A² wird gewählt aus C₁-C₅₀-Alkylen, substituiert oder nicht substituiert, wobei eine oder mehrere nicht-benachbarte CH₂-Gruppen durch Sauerstoff ersetzt sein können.

12. Verfahren zur Herstellung von (Co)polymeren nach einem der Ansprüche 1 bis 6, **dadurch gekennzeichnet, dass** man mindestens ein (Co)monomer der allgemeinen Formel IV a oder IV b 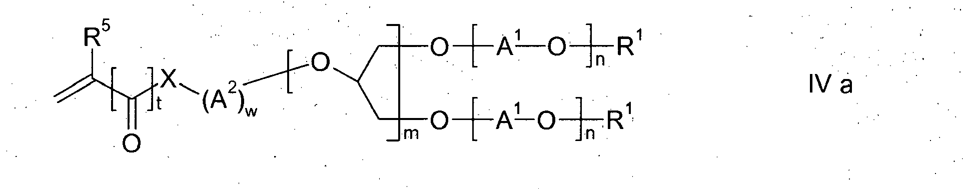 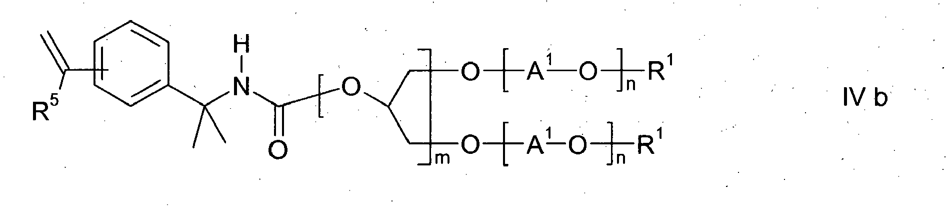 radikalisch polymerisiert, gegebenenfalls in Gegenwart von mindestens einem Comonomer der allgemeinen Formel V a bis V c 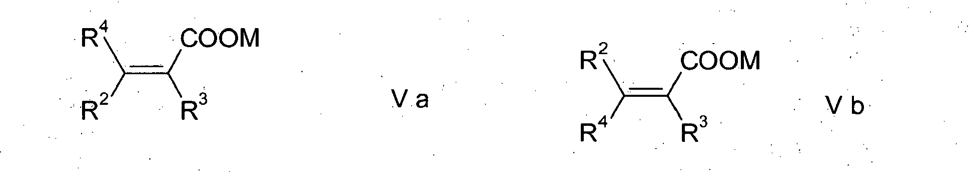 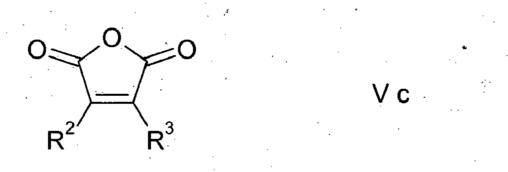 wobei die Variablen wie folgt definiert sind:
R², R³ gleich oder verschieden und gewählt aus C₁-C₆-Alkyl und Wasserstoff,
R⁴ C₁-C₆-Alkyl, COOM oder Wasserstoff,
M gewählt aus Ammonium, substituiert oder unsubstituiert, Wasserstoff und Metallkationen,
R⁵ gewählt aus Wasserstoff und Methyl,
t gewählt aus null und eins,
w gewählt aus null und eins,
X gewählt aus einer Einfachbindung, Sauerstoff und N-H,
A² gewählt aus C₁-C₅₀-Alkylen, substituiert oder nicht substituiert, wobei eine oder mehrere nicht-benachbarte CH₂-Gruppen durch Sauerstoff ersetzt sein können.

13. Verbindung der allgemeinen Formel IV a 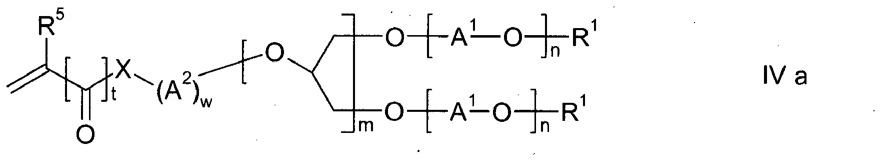 in der die Variablen wie folgt definiert sind:
R¹ verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
A¹ verschieden oder gleich und gewählt aus C₂-C₁₀-Alkylen, C₆-C₁₀-Arylen und C₇-C₁₀-Aralkylen,
n verschieden oder gleich und gewählt aus null bis 200,
m verschieden oder gleich und gewählt aus 1 bis 6,
t gewählt aus null und eins,
w gewählt aus null und eins,
X gewählt aus einer Einfachbindung, Sauerstoff und N-H,
A² gewählt aus C₁-C₅₀-Alkylen, substituiert oder nicht substituiert, wobei eine oder mehrere nicht-benachbarte CH₂-Gruppen durch Sauerstoff ersetzt sein können,
R⁵ gewählt aus Wasserstoff und Methyl.

14. Verbindung der allgemeinen Formel IV b 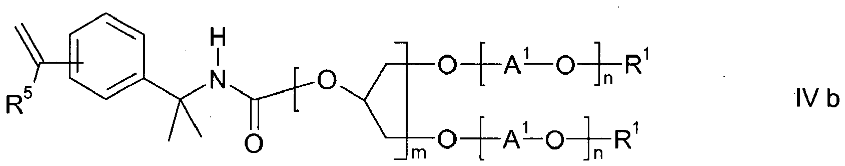 in der die Variablen wie folgt definiert sind:
R¹ verschieden oder gleich und gewählt aus Wasserstoff, C₁-C₃₀-Alkyl, C₃-C₁₀-Cycloalkyl, C₆-C₃₀-Aryl und C₇-C₃₀-Aralkyl,
A¹ verschieden oder gleich und gewählt aus C₂-C₁₀-Alkylen, C₆-C₁₀-Arylen und C₇-C₁₀-Aralkylen,
n verschieden oder gleich und gewählt aus null bis 200,
m verschieden oder gleich und gewählt aus 1 bis 6,
R⁵ gewählt aus Wasserstoff und Methyl.

## Claims

1. A copolymer comprising as comonomers in copolymerized form
(A) at least one ethylenically unsaturated mono- or dicarboxylic acid,
(B) at least one ethylenically unsaturated compound which has at least one structural unit of the general formula I per molecule: 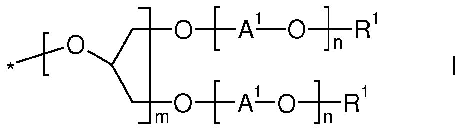 where the variables are defined as follows:
R¹ is different or identical and selected from hydrogen, C₁-C₃₀-alkyl, C₃-C₁₀-cycloalkyl, C₆-C₃₀-aryl and C₇-C₃₀-aralkyl,
A¹ is different or identical and selected from C₂-C₁₀-alkylene, C₆-C₁₀-arylene and C₇-C₁₀-aralkylene,
n is different or identical and selected from zero to 200,
m is different or identical and selected from 1 to 6, (C) at least one further comonomer.

2. The copolymer according to claim 1, which has at least one structural unit of the general formula II 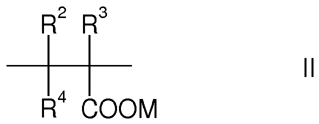 where the variables are defined as follows:
R², R³ are identical or different and selected from C₁-C₆-alkyl and hydrogen,
R⁴ is C₁-C₆-alkyl, COOM or hydrogen,
M is selected from ammonium, substituted or unsubstituted, hydrogen and metal cations.

3. The copolymer according to claim 1 or 2, wherein compound (B) has a molecular weight M_{w} in the range from 200 to 100 000 g/mol.

4. The copolymer according to any one of claims 1 to 3, wherein compound (B) has a K value in accordance with Fikentscher in the range from 8 to 40, measured in water/THF mixtures.

5. The copolymer according to any one of claims 1 to 4, wherein the molar ratio of structural units of the formula I to structural units of the formula II is in the range from 0.01 to 10.

6. The copolymer according to any one of claims 1 to 5, wherein at least one structural unit of the general formula I is bonded to the basic backbone of said copolymer via a group of the formula III a 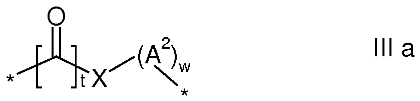 or III b 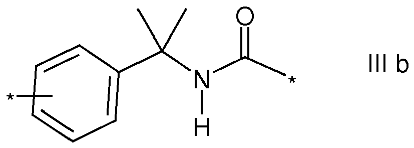 where the variables are defined as follows:
X is selected from a single bond, oxygen and N-H,
t is selected from zero and one,
w is selected from zero and one,
A² is selected from C₁-C₅₀-alkylene, substituted or unsubstituted, where one or more nonadjacent CH₂ groups may be replaced by oxygen.

7. The use of copolymers according to any one of claims 1 to 6 as thickeners.

8. A detergent or cleaner or cosmetic preparation comprising at least one copolymer according to any one of claims 1 to 6.

9. A compound (B), which has at least one structural unit of the general formula I per molecule: 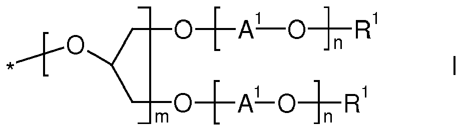 where the variables are defined as follows:
R¹ is different or identical and selected from hydrogen, C₁-C₃₀-alkyl, C₃-C₁₀-cycloalkyl, C₆-C₃₀-aryl and C₇-C₃₀-aralkyl,
A¹ is different or identical and selected from C₂-C₁₀-alkylene, C₆-C₁₀-arylene and C₇-C₁₀-aralkylene,
n is different or identical and selected from zero to 200,
m is different or identical and selected from 1 to 6.

10. The compound (B) according to claim 9, which has a molecular weight M_{w} in the range from 200 to 100 000 g/mol.

11. The compound (B) according to claim 9 or 10, which comprises, in copolymerized form, at least one comonomer of the general formula IV a or IV b, 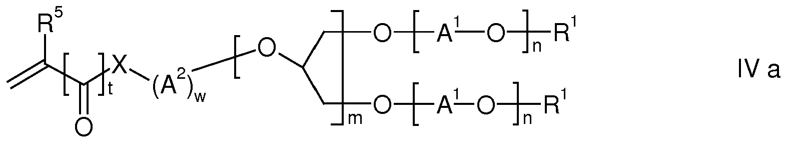 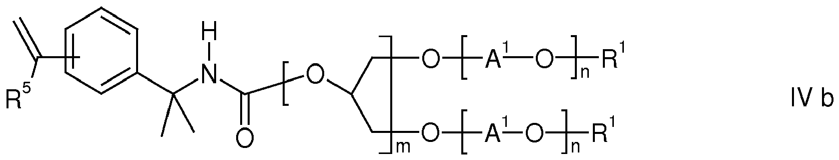 where the variables are defined as follows:
R¹ is different or identical and selected from hydrogen, C₁-C₃₀-alkyl, C₃-C₁₀-cycloalkyl, C₆-C₃₀-aryl and C₇-C₃₀-aralkyl,
A¹ is different or identical and selected from C₂-C₁₀-alkylene, C₆-C₁₀-arylene and C₇-C₁₀-aralkylene,
n is different or identical and selected from zero to 200,
m is different or identical and selected from 1 to 6,
R⁵ is selected from hydrogen and methyl,
t is selected from zero and one,
w is selected from zero and one,
X is selected from a single bond, oxygen and N-H,
A² is selected from C₁-C₅₀-alkylene, substituted or unsubstituted, where one or more nonadjacent CH₂ groups may be replaced by oxygen.

12. A method of preparing (co)polymers according to any one of claims 1 to 6, wherein at least one (co)monomer of the general formula IV a or IV b 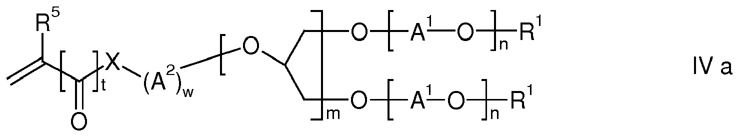 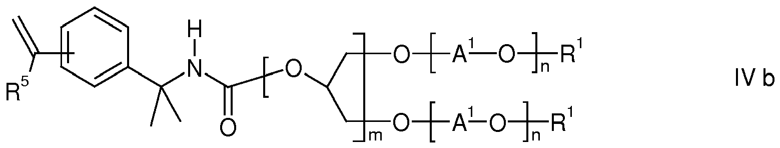 is free-radically polymerized, optionally in the presence of at least one comonomer of the general formula V a to V c 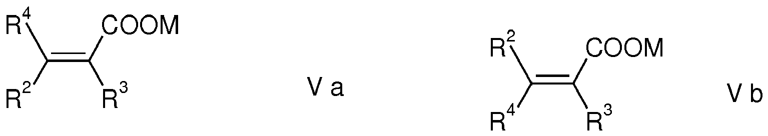 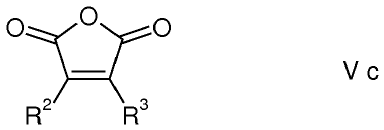 where the variables are defined as follows:
R², R³ are identical or different and selected from C₁-C₆-alkyl and hydrogen,
R⁴ is C₁-C₆-alkyl, COOM or hydrogen,
M is selected from ammonium, substituted or unsubstituted, hydrogen and metal cations,
R⁵ is selected from hydrogen and methyl,
t is selected from zero and one,
w is selected from zero and one,
X is selected from a single bond, oxygen and N-H,
A² is selected from C₁-C₅₀-alkylene, substituted or unsubstituted, where one or more nonadjacent CH₂ groups may be replaced by oxygen.

13. A compound of the general formula IV a 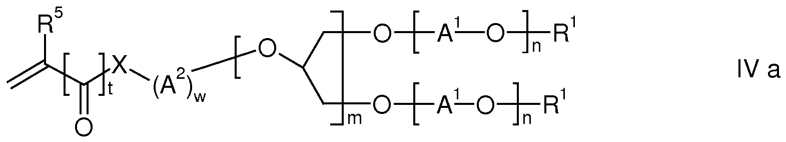 in which the variables are defined as follows:
R¹ is different or identical and selected from hydrogen, C₁-C₃₀-alkyl, C₃-C₁₀-cycloalkyl, C₆-C₃₀-aryl and C₇-C₃₀-aralkyl,
A¹ is different or identical and selected from C₂-C₁₀-alkylene, C₆-C₁₀-arylene and C₇-C₁₀-aralkylene,
n is different or identical and selected from zero to 200,
m is different or identical and selected from 1 to 6,
t is selected from zero and one,
w is selected from zero and one,
X is selected from a single bond, oxygen and N-H,
A² is selected from C₁-C₅₀-alkylene, substituted or unsubstituted, where one or more nonadjacent CH₂ groups may be replaced by oxygen,
R⁵ is selected from hydrogen and methyl.

14. A compound of the general formula IV b 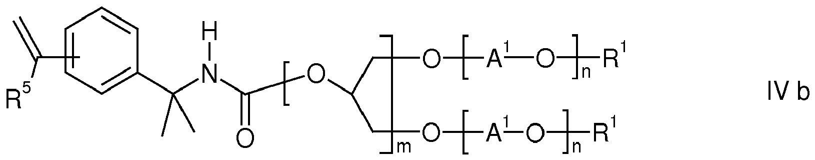 in which the variables are defined as follows:
R¹ is different or identical and selected from hydrogen, C₁-C₃₀-alkyl, C₃-C₁₀-cycloalkyl, C₆-C₃₀-aryl and C₇-C₃₀-aralkyl,
A¹ is different or identical and selected from C₂-C₁₀-alkylene, C₆-C₁₀-arylene and C₇-C₁₀-aralkylene,
n is different or identical and selected from zero to 200,
m is different or identical and selected from 1 to 6,
R⁵ is selected from hydrogen and methyl.

## Revendications

1. Copolymère, contenant en tant que comonomère incorporé par polymérisation
(A) au moins un acide mono- ou dicarboxylique à insaturation éthylénique,
(B) au moins un composé à insaturation éthylénique, qui contient par molécule au moins un motif structural de formule générale I : 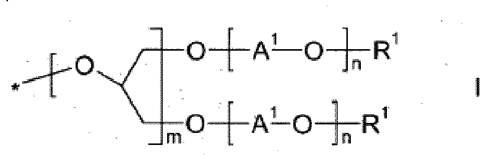 les variables étant définies comme suit :
R¹ est le même ou différent et choisi parmi un atome d'hydrogène, des groupes alkyle en C₁-C₃₀, cycloalkyle en C₃-C₁₀, aryle en C₆-C₃₀ et aralkyle en C₇-C₃₀,
A¹ est le même ou différent et choisi parmi des groupes alkylène en C₂-C₁₀, arylène en C₆-C₁₀ et aralkylène en C₇-C₁₀,
n est le même ou différent et choisi parmi zéro à 200,
m est le même ou différent et choisi parmi 1 à 6, (C) au moins un autre comonomère.

2. Copolymère selon la revendication 1, **caractérisé en ce qu'**il comporte au moins un motif structural de formule générale II, 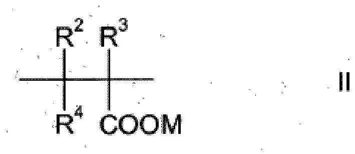 les variables étant définies comme suit :
R², R³ sont identiques ou différents et choisis parmi un groupe alkyle en C₁-C₆ et un atome d'hydrogène,
R⁴ est un groupe alkyle en C₁-C₆, COOM ou un atome d'hydrogène,
M est choisi parmi un ion ammonium, substitué ou non substitué, un atome d'hydrogène et des cations métalliques.

3. Copolymère selon la revendication 1 ou 2, **caractérisé en ce que** le composé (B) présente une masse moléculaire M_{w} dans la plage de 200 à 100 000 g/mole.

4. Copolymère selon l'une quelconque des revendications 1 à 3, **caractérisé en ce que** le composé (B) présente un indice K selon Fikentscher dans la plage de 8 à 40, mesuré dans des mélanges eau/THF.

5. Copolymère selon l'une quelconque des revendications 1 à 4, **caractérisé en ce que** le rapport molaire des motifs structuraux de formule I aux motifs structuraux de formule II se situe dans la plage de 0,01 à 10.

6. Copolymère selon l'une quelconque des revendications 1 à 5, **caractérisé en ce qu'**au moins un motif structural de formule générale I est lié au squelette de base dudit copolymère par un groupe de formule III a 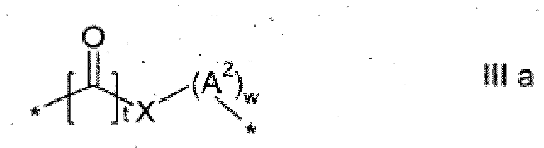 ou III b 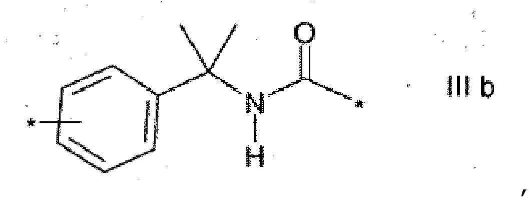 les variables étant définies comme suit :
X est choisi parmi une simple liaison, un atome d'oxygène et N-H,
t est choisi parmi zéro et un,
w est choisi parmi zéro et un,
A² est choisi parmi des groupes alkylène en C₁-C₅₀, substitués ou non substitués, un ou plusieurs groupes CH₂ non contigus pouvant être remplacés par un atome d'oxygène.

7. Utilisation de copolymères selon l'une quelconque des revendications 1 à 6 en tant qu'épaississant.

8. Produits de lavage et de nettoyage ou préparations cosmétiques, contenant au moins un copolymère selon l'une quelconque des revendications 1 à 6.

9. Composés (B), **caractérisés en ce qu'**ils comportent par molécule au moins un motif structural de formule générale I : 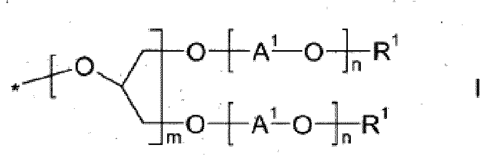 les variables étant définies comme suit :
R¹ est le même ou différent et choisi parmi un atome d'hydrogène, des groupes alkyle en C₁-C₃₀, cycloalkyle en C₃-C₁₀, aryle en C₆-C₃₀ et aralkyle en C₇-C₃₀,
A¹ est le même ou différent et choisi parmi des groupes alkylène en C₂-C₁₀, arylène en C₆-C₁₀ et aralkylène en C₇-C₁₀,
n est le même ou différent et choisi parmi zéro à 200,
m est le même ou différent et choisi parmi 1 à 6.

10. Composés (B) selon la revendication 9, **caractérisés en ce qu'**ils présentent une masse moléculaire M_{w} dans la plage de 200 à 100 000 g/mole.

11. Composés (B) selon la revendication 9 ou 10, **caractérisés en ce qu'**ils contiennent incorporé par polymérisation au moins un comonomère de formule générale IV a ou IV b, 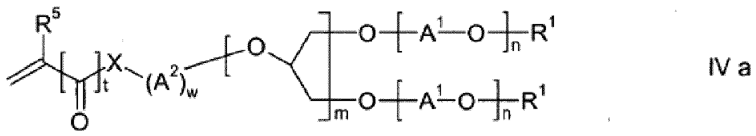 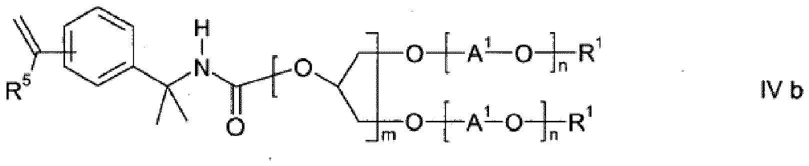 les variables étant définies comme suit :
R¹ est le même ou différent et choisi parmi un atome d'hydrogène, des groupes alkyle en C₁-C₃₀, cycloalkyle en C₃-C₁₀, aryle en C₆-C₃₀ et aralkyle en C₇-C₃₀,
A¹ est le même ou différent et choisi parmi des groupes alkylène en C₂-C₁₀, arylène en C₆-C₁₀ et aralkylène en C₇-C₁₀,
n est le même ou différent et choisi parmi zéro à 200,
m est le même ou différent et choisi parmi 1 à 6,
R⁵ est choisi parmi un atome d'hydrogène et le groupe méthyle,
t est choisi parmi zéro et un, w est choisi parmi zéro et un,
X est choisi parmi une simple liaison, un atome d'oxygène et N-H,
A² est choisi parmi des groupes alkylène en C₁-C₅₀, substitués ou non substitués, un ou plusieurs groupes CH₂ non contigus pouvant être remplacés par un atome d'oxygène.

12. Procédé pour la préparation de (co)polymères selon l'une quelconque des revendications 1 à 6, **caractérisé en ce qu'**on polymérise par voie radicalaire au moins un (co)monomère de formule générale IV a ou IV b 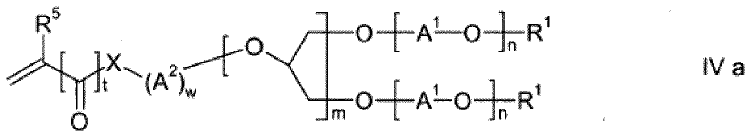 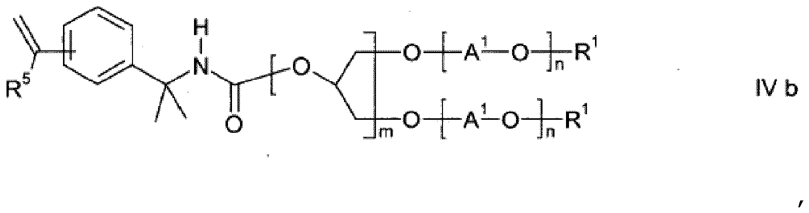 éventuellement en présence d'au moins un comonomère de formule générale V a à V c 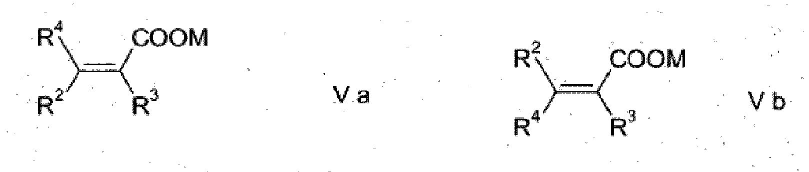 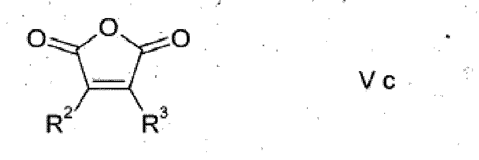 les variables étant définies comme suit :
R², R³ sont identiques ou différents et choisis parmi un groupe alkyle en C₁-C₆ et un atome d'hydrogène,
R⁴ est un groupe alkyle en C₁-C₆, COOM ou un atome d'hydrogène,
M est choisi parmi un ion ammonium, substitué ou non substitué, un atome d'hydrogène et des cations métalliques,
R⁵ est choisi parmi un atome d'hydrogène et le groupe méthyle,
t est choisi parmi zéro et un,
w est choisi parmi zéro et un,
X est choisi parmi une simple liaison, un atome d'oxygène et N-H,
A² est choisi parmi des groupes alkylène en C₁-C₅₀, substitués ou non substitués, un ou plusieurs groupes CH₂ non contigus pouvant être remplacés par un atome d'oxygène.

13. Composé de formule générale IV a 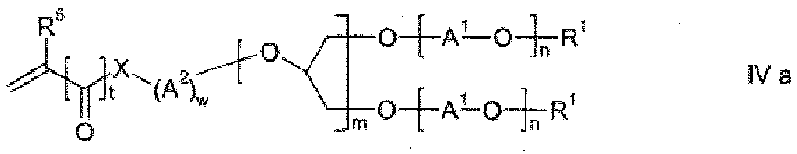 dans laquelle les variables sont définies comme suit :
R¹ est le même ou différent et choisi parmi un atome d'hydrogène, des groupes alkyle en C₁-C₃₀, cycloalkyle en C₃-C₁₀, aryle en C₆-C₃₀ et aralkyle en C₇-C₃₀,
A¹ est le même ou différent et choisi parmi des groupes alkylène en C₂-C₁₀, arylène en C₆-C₁₀ et aralkylène en C₇-C₁₀,
n est le même ou différent et choisi parmi zéro à 200,
m est le même ou différent et choisi parmi 1 à 6,
t est choisi parmi zéro et un,
w est choisi parmi zéro et un,
X est choisi parmi une simple liaison, un atome d'oxygène et N-H,
A² est choisi parmi des groupes alkylène en C₁-C₅₀, substitués ou non substitués, un ou plusieurs groupes CH₂ non contigus pouvant être remplacés par un atome d'oxygène,
R⁵ est choisi parmi un atome d'hydrogène et le groupe méthyle.

14. Composé de formule générale IV b 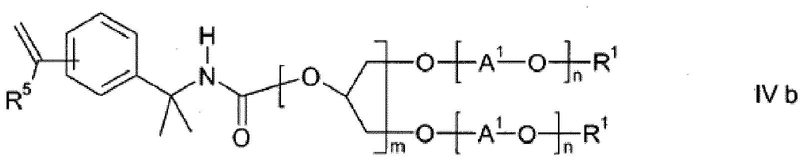 dans laquelle les variables sont définies comme suit :
R¹ est le même ou différent et choisi parmi un atome d'hydrogène, des groupes alkyle en C₁-C₃₀, cycloalkyle en C₃-C₁₀, aryle en C₆-C₃₀ et aralkyle en C₇-C₃₀,
A¹ est le même ou différent et choisi parmi des groupes alkylène en C₂-C₁₀, arylène en C₆-C₁₀ et aralkylène en C₇-C₁₀,
n est le même ou différent et choisi parmi zéro à 200,
m est le même ou différent et choisi parmi 1 à 6,
R⁵ est choisi parmi un atome d'hydrogène et le groupe méthyle.
